Europäisches Patentamt

European Patent Office

Office européen des brevets

(11) Publication number: **0 237 966**
**A2**

## (12) EUROPEAN PATENT APPLICATION

(21) Application number: 87103601.8

(22) Date of filing: 12.03.87

(51) Int. Cl.4: **C12N 15/00** , **C12P 21/02** , **C07K 13/00** , **C07K 3/20**

The applicant has filed a statement in accordance with Rule 28 (4) EPC (issue of a sample only to an expert). Accession number(s) of the deposit(s): IFO 14494, IFO 14532, IFO 50092, FERM BP-1280, FERM BP-1281.

(30) Priority: 14.03.86 JP 57919/86
09.04.86 JP 82699/86
29.09.86 JP 231428/86

(43) Date of publication of application:
23.09.87 Bulletin 87/39

(84) Designated Contracting States:
AT BE CH DE ES FR GB GR IT LI LU NL SE

(71) Applicant: **Takeda Chemical Industries, Ltd.**
**27, Doshomachi 2-chome Higashi-ku**
**Osaka-shi Osaka, 541(JP)**

(72) Inventor: **Kurokawa, Tsumotu**
**1-50, Suimeidai 1-chome**
**Kawanishi Hyogo 666-01(JP)**
Inventor: **Sasada, Reiko**
**C1-405, 2 Takenodai**
**Nagaokakyo Kyoto 560(JP)**
Inventor: **Iwane, Makoto**
**50-1, Yamadaminami**
**Suita Osaka 565(JP)**
Inventor: **Igarashi, Koichi**
**66-3, Shimogamo Miyazaki-cho Sakyo-ku**
**Kyoto 606(JP)**

(74) Representative: **von Kreisler, Alek,**
**Dipl.-Chem. et al**
**Deichmannhaus am Hauptbahnhof**
**D-5000 Köln 1(DE)**

(54) **Human basic fibroblast growth factor.**

(57) A human basic fibroblast growth factor (hbFGF), which is usable for a cure promoter for burns etc., therapeutic agent for thrombosis etc. or a reagent for promoting cell cultivation, can be produced in a large quantity by cultivating a transformant as transformed with a vector including a DNA coding the hbFGF.

EP 0 237 966 A2

## POLYPEPTIDE, DNA AND ITS USE

This invention relates to a human basic fibroblast growth factor (hereinafter sometimes referred to as "hbFGF") and to a method for producing the hbFGF by a genetic engineering technique.

The basic fibroblast growth factor (bFGF) is a basic polypeptide hormone which is secreted mainly from the pituitary gland and has a molecular weight of about 17,000. It was first isolated as a factor showing potent growth promoting action on fibroblasts such as BALB/c3T3 cells [D. Gospodarowicz: Nature, 249, 123 (1974)]. Later, however, it was revealed that it exhibits growth-promoting action on almost all mesoderm-derived cells [D. Gospodarowicz et al.: National Cancer Institute Monograph, 48, 109 (1978)]. The vascularizing activity of bFGF, among others, conjointly with its cell growth promoting activity, suggests the possibility of its use as a therapeutic agent for lesions and as a preventive and therapeutic agent for thrombosis, arteriosclerosis and the like. However, the quantity of naturally occurring hbFGF is very slight and attempts to obtain this factor from human tissues have encountered serious difficulties arising from various restrictions and limitations. Accordingly, the amino acid sequence of hbFGF has not been determined as yet.

On the other hand, animal-derived bFGF, for which the material required is available comparatively readily, has already been obtained in a purified form from the bovine pituitary gland, for instance, and its amino acid sequence has been determined [F. Esch et al.: Proceedings of the National Academy of Sciences of the Untied States of America (Proc. Natl. Acad. Sci. USA), 82, 6507 (1985)]. However, in this case, too, large scale production is very difficult.

As mentioned above, much remains unknown of the properties and amino acid sequence of hbFGF and the relevant gene. Therefore, for enabling its use as a drug, an earnest desire has been felt to identify the gene coding for the factor and develop a method for producing the protein on a large scale by utilizing the genetic engineering technology.

Generally, proteins derived from animals close to humans show very high homology with respect to their amino acid sequences and, in most cases, the differential amino acids are derived by one point mutation relative to the codons. Therefore, it is presumable that the DNA sequence derivable from the amino acid sequence of the above-mentioned bovine bFGF should be very similar in part to the DNA sequence of the hbFGF gene although a number of DNA sequences might be possible due to degeneration of codons. Based on such assumption, the present inventors synthesized part of the bFGF gene having the possible nucleotide sequences, cloned the hbFGF cDNA from human cells using said part of the bFGF gene as a DNA probe and constructed a recombinant DNA involving said hbFGF cDNA. It was thus found that cultivation of a transformant transformed with said DNA can result in production of hbFGF. The present inventors conducted further researches on the basis of these findings and, as a result, have now completed the present invention.

Thus, the present invention provides:

(I) A human basic fibroblast growth factor (II) which is a polypeptide including the amino acid sequence:

Pro-Ala-Leu-Pro-Glu-Asp-Gly-Gly-Ser-Gly-Ala-Phe-Pro-Pro-Gly-His-Phe-Lys-Asp-Pro-Lys-Arg-Leu-Tyr-Cys-Lys-Asn-Gly-Gly-Phe-Phe-Leu-Arg-Ile-His-Pro-Asp-Gly-Arg-Val-Asp-Gly-Val-Arg-Glu-Lys-Ser-Asp-Pro-His-Ile-Lys-Leu-Gln-Leu-Gln-Ala-Glu-Glu-Arg-Gly-Val-Val-Ser-Ile-Lys-Gly-Val-Cys-Ala-Asn-Arg-Tyr-Leu-Ala-Met-Lys-Glu-Asp-Gly-Arg-Leu-Leu-Ala-Ser-Lys-Cys-Val-Thr-Asp-Glu-Cys-Phe-Phe-Phe-Glu-Arg-Leu-Glu-Ser-Asn-Asn-Tyr-Asn-Thr-Tyr-Arg-Ser-Arg-Lys-Tyr-Thr-Ser-Trp-Tyr-Val-Ala-Leu-Lys-Arg-Thr-Gly-Gln-Tyr-Lys-Leu-GlY-Ser-Lys-Thr-Gly-Pro-Gly-Gln-Lys-Ala-Ile-Leu-Phe-Leu-Pro-Met-Ser-Ala-Lys-Ser( I )

(2) A recombinant DNA (III) which contains a base sequence coding for the human basic fibroblast growth factor (II);

(3) A transformant as transformed as a host cell with a vector including the DNA (III);

(4) A method for producing a transformant, which comprises subjecting a host cell to transformation with a vector including the DNA (III);

(5) A method for producing the human basic fibroblast growth factor (II), which comprises cultivating a transformant of said item (3) in a medium and recovering the factor as produced and accumulated in the cultured broth;

(6) A substantially pure human basic fibroblast growth factor protein, the factor being obtained by genetic engineering technique; and

(7) A method for producing a substantially pure human basic fibroblast growth factor protein, which comprises subjecting a basic fibroblast growth factor obtained by a genetic engineering technique to a purification procedure of high performance liquid chromatography using a heparin column.

The hbFGF (II) according to this invention contains at least the polypeptide of formula (I).

The above DNA (III) preferably has a base sequence (V) containing, among others, the base sequence:

```
C C C G C C T T G C C C G A G G A T G G C G G C A G C G G C G C C T T C C C G C C C G G C C
A C T T C A A G G A C C C C A A G C G G C T G T A C T G C A A A A A C G G G G G C T T C T T C
C T G C G C A T C C A C C C C G A C G G C C G A G T T G A C G G G G T C C G G G A G A A G A
G C G A C C C T C A C A T C A A G C T A C A A C T T C A A G C A G A A G A G A G A G G A G T T
G T G T C T A T C A A A G G A G T G T G T G C T A A C C G T T A C C T G G C T A T G A A G G A
A G A T G G A A G A T T A C T G G C T T C T A A A T G T G T T A C G G A T G A G T G T T T C T T
T T T T G A A C G A T T G G A A T C T A A T A A C T A C A A T A C T T A C C G G T C A A G G A A
A T A C A C C A G T T G G T A T G T G G C A C T G A A A C G A A C T G G G C A G T A T A A A C T
T G G A T C C A A A A C A G G A C C T G G G C A G A A A G C T A T A C T T T T T C T T C C A A T
G T C T G C T A A G A G A G C (IV)
```

Preferred as the hbFGF concerned with the DNA, transformant and production method according this invention is the hbFGF (II) as represented by the amino acid sequence given above.

An expression vector which contains a DNA having a base sequence coding for the polypeptide of the hbFGF protein and is to be used in carrying out the method of this invention can be produced, for example, by:

(a) Isolating an RNA coding for hbFGF;

(b) Synthesizing a single-stranded complementary DNA (cDNA) based on said RNA and then synthesizing the corresponding double-stranded DNA;

(c) Inserting said complementary DNA into a plasmid;

(d) Transforming a host with the resultant recombinant plasmid;

(e) Cultivating the transformant obtained and isolating that plasmid which contains the DNA as desired from the transformant by an appropriate method, for example by the colony hybridization method using a DNA probe;

(f) Excising the cloned, desired DNA, from said plasmid; and

(g) Inserting said cloned DNA into a vehicle at a site downstream from a promoter.

RNAs coding for hbFGF can be obtained from a variety of hbFGF-producing cells, for example human pituitary-derived cells or human fibroblasts. As said human fibroblasts, there may be mentioned WI38 - (ATCC No. CCL-75) and IMR90 (ATCC No. CCL-186), among others. Said cell lines WI38 and IMR90 are listed on the Catalogue of Cell Lines & Hybridomas, 5th edition, 1985, published by The American Type Culture Collection.

As a method for RNA preparation from hbFGF-producing cells, there may be mentioned the guanidine thiocyanate method [J. M. Chirgwin et al.: Biochemistry, 18, 5294 (1979)], for instance.

Using the thus-obtained RNA as the template together with reverse transcriptase, a cDNA is synthesized by the method of H. Okayama et al. [Molecular and Cellular Biology, 2, 161 (1982)], for instance, and the cDNA obtained is inserted into a plasmid.

The plasmid into which said cDNA is to be inserted is, for example, a plasmid derived from Escherichia coli such as pBR322 [Gene, 2, 95 (1977)], pBR325 [Gene, 4 , 121 (1978)], pUC12 [Gene, 19, 259 (1982)] or pUC13 [Gene, 19, 259 (1982)], or one derived from Bacillus subtilis such as pUB110 [Biochemical and Biophysical Research Communications, 112, 678 (1983)]. Any other plasmids capable of being replicated and maintained within the host employed may be used as well.

As a method for insertion into a plasmid, there may be mentioned the method described by T. Maniatis et al. in Molecular Cloning, Cold Spring Harbor Laboratory, page 239 (1982), among others.

The plasmid with the above cDNA inserted therein may be a plasmid obtained by using the cDNA library produced by inserting cDNAs synthesized on the basis of mRNAs derived from normal human diploid cells into the pCD vector [cf. Okayama et al.: Molecular and Cellular Biology, 3, 280 (1983)] with Escherichia coli x1776 being used as the host [such library being available from Dr. Okayama at the National Institute of Child Health and Human Development, Bethesda, USA).

The plasmid obtained in this manner is introduced into an appropriate host, for example a bacterial strain belonging to the genus Escherichia or Bacillus.

Examples of the above strain of the genus Escherichia are Escherichia coli K12DH1 [Proc. Natl. Acad. Sci. USA, 60, 160 (1968)], M103 [Nucleic Acids Research, 9, 309 (1981)], JA221 [Journal of Molecular Biology, 120, 517 (1978)], HB101 [Journal of Molecular Biology, 41, 459 (1969)] and C600 [Genetics, 39, 440 (1954)].

Examples of the above strain of the genus Bacillus are Bacillus subtilis M1114 [Gene, 24, 255 (1983)] and 207-21 [Journal of Biochemistry, 95, 87 (1984)].

As a method for effecting transformation, there may be mentioned the calcium chloride or calcium chloride/rubidium chloride method described by T. Maniatis et al. in Molecular Cloning, Cold Spring Harbor Laboratory, page 249 (1982), among others.

From among the thus-obtained transformants, the desired clones are selected by a per se known method, for example by the colony hybridization method [Gene, 10, 63 (1980)] plus the DNA base sequence determination method [Proc. Natl. Acad. Sci. USA, 74 , 560 (1977); Nucleic Acids Research, 9, 309 (1981)].

In this way, a microorganism which carries a vector having a cloned DNA containing an hbFGF-encoding base se quence is obtained.

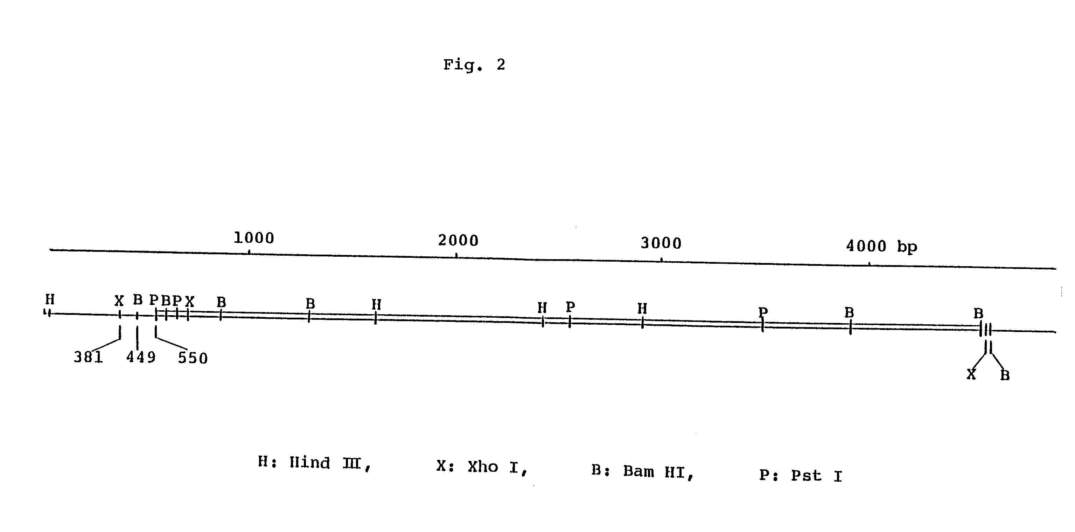

The plasmid pTB627 harbored by the transformant Escherichia coli KI2DHI/pTB627 obtained in Example I (I) to be described later herein has a DNA containing an hbFGF (II)-encoding base sequence. The restriction enzyme cleavage sites on said DNA are shown in Fig. 2. As shown in Fig. 2, said DNA contains about 4,000 base pairs and is cleaved into 4 fragments by the restriction enzyme PstI or Hind III. The base sequence coding for hbFGF (II) resides on the most upstream HindIII fragment (on the left end in Fig. 2).

The plasmid is isolated from said microorganism.

For such plasmid isolation, the alkaline extraction method [H. C. Birnboim et al.: Nucleic Acids Research, I, 1513 (1979)], for instance, may be used.

The above-mentioned plasmid having the cloned DNA containing the hbFGF-encoding base sequence is used as it is or, as desired, subjected to restriction enzyme treatment for excision of said DNA.

Expression vectors can be obtained by inserting the cloned cDNA into a vehicle (vector) suited for expression of said cDNA at a site downstream of a promoter.

Said vector includes, among others, the above-mentioned Escherichia coli-derived plasmids (e.g. pBR322, pBR325, pUCI2, pUCI3) and Bacillus subtilis -derived plasmids (e.g. pUBII0, pTP5, pCI94) as well as yeast-derived plasmids (e.g. pSHI9, pSHI5), bacteriophages such as λ phage, and animal viruses such as retroviruses and vaccinia virus.

Said cDNA may have ATG as the translational start codon at its 5′ end. It may also have TAA, TGA or TAG as a translational termination codon at the 3′ end. For effecting expression of said cDNA , a promoter is connected thereto at a site upstream from said cDNA. The promoter to be used in the practice of the invention may be any promoter if it is appropriate and adapted for the host employed for the expression of said cDNA.

When the host to be transformed is a strain belonging to the genus Escherichia, the trp promoter, lac promoter, rec A Promoter, λPL promoter and lpp promoter are preferred among others. When the host is a strain of the genus Bacillus, the SPOI promoter, SPO2 promoter and penP promoter, for instance, are preferred. When the host is a yeast strain, the PHO5 promoter, PGK promoter, GAP promoter and ADH promoter are preferred amongst others. In particular, it is preferable that the host should be a strain of the genus Escherichia and that the promoter should be the trp promoter or λPL promoter.

When the host is an animal cell line, SV40-derived promoters and retrovirus promoters are usable among others. In particular, SV40-derived promoters are preferable.

The thus-constructed vector containing the DNA (III) is used to produce transformants.

Examples of the host include strains belonging to the genus Escherichia, strains belonging to the genus Bacillus, yeasts and animal cells. Representative examples of the strains of the genera Escherichia and Bacillus are those mentioned hereinbefore.

As the yeasts, there may be mentioned Saccharomyces cerevisiae AH22R⁻, NA87-IIA and DKD-5D, for instance.

As the animal cells, cell lines are preferred, and there may be mentioned monkey COS-7 [Gluzman, Y, cell 23, 157 (1981)] and Vero cells, Chinese hamster CHO cells, mouse L cells and human FL cells, among others.

The transformation of the above-strains of the genus Escherichia is conducted by the method described in Proc. Natl. Acad. Sci. USA, 69, 2110 (1972) or in Gene, 17, 107 (1982), for instance.

The transformation of a strain of the genus Bacillus is performed, for example, by the method described in Molecular and General Genetics, 168, 111 (1979).

The transformation of yeasts is carried out, for example, by the method described in Proc. Natl. Acad. Sci. USA, 75, 1929 (1978).

The transformation of animal cells is conducted by the method described in Virology, 52, 456 (1973), among others.

In this manner, there are obtained transformants as transformed with vectors containing the DNA (II).

The medium to be used in cultivating a transformant obtained with a strain of the genus Escherichia or Bacillus as the host is suitably a liquid one which contains substances required for the growth of said transformant, for example carbon and nitrogen sources and inorganic nutrients. Glucose, dextrin, soluble starch and sucrose, for instance, may serve as carbon sources. Ammonium salts. nitrate salts, corn steep liquor, peptone, casein, meat extract, soybean cake and potato extract may serve as nitrogen sources. As the inorganic nutrients, there may be mentioned calcium chloride, sodium dihydrogen phosphate and magnesium chloride, among others. Yeast extracts, vitamins, growth promoters like may further be added.

The medium should preferably have a pH of about 6 to 8.

M9 medium containing glucose and casamino acids (Miller: Journal of Experiments in Molecular Genetics, 431-433, Cold Spring Harbor Laboratory, New York, 1972), for instance, is a preferable medium for use in cultivating microorganism of the genus Escherichia . For efficient performance of the promoter function, such an agent as 3-β-indolylacrylic acid in the case of trp promoter, for instance, may be added as necessary.

When the host is a microorganism of the genus Escherichia, the cultivation is conducted generally at about 15 to 43°C for about 3 to 24 hours. If necessary, aeration and/or stirring may be made.

When the host is a microorganism of the genus Bacillus, the cultivation is performed generally at about 30 to 40°C for about 6 to 24 hours. Aeration and/or stirring may be conducted as necessary.

When the host is a yeast transformant, Burkholder's minimum medium [Bostian, K. L. et al.: Proc. Natl. Acad. Sci. USA, 77 , 4505 (1980)], for instance, may be used as the medium. The pH of the medium should preferably be adjusted to about 5 to 8. The cultivation is carried out generally at about 20 to 35°C for about 24 to 72 hours, with aeration and/or stirring as necessary.

As the medium to be used in cultivating an animal cell transformant, there may be mentioned, for example, MEM medium [Science, 122, 501 (1952)], DMEM medium [Virology, 8, 396 (1959)], RPMI 1640 medium [Journal of the American Medical Association, 199, 519 (1967)] or 199 medium [Proceedings of the Society for the Biological Medicine, 73 , 1 (1950)], which is further added about 5 to 20% of felal calf serum. The medium should preferably have a pH of about 6 to 8. The cultivation is carried out generally at about 30 to 40°C for about 15 to 60 hours, with aeration and/or stirring as necessary.

The hbFGF protein can be isolated in a purified form from the above cultivation product, for example by the following methods:

In extracting the hbFGF protein from cultured cells, said cells after cultivation are collected by a known method and then processed by an appropriate method such as the method comprising suspending the cells in a buffer solution containing a protein-denaturing agent such as guanidine hydrochloride to thereby cause extracellular dissolution of the desired protein or the method comprising disrupting the cells by French press, sonication, lysozyme treatment and/or freezing and thawing, followed by centrifugation for the recovery of the hbFGF protein. The French press treatment or combined use of lysozyme treatment and sonication are particularly preferred.

For the purification of the hbFGF protein from the supernatant obtained in the above manner, appropriate combinations of known isolation and purification methods can be used. As such known isolation and purification methods, there may be mentioned the methods utilizing solubility differences, such as the salting-out method and solvent precipitation method, the methods utilizing molecular weight differences in the main, such as the dialysis method, ultra-filtration method, gel filtration method and SDS-polyacrylamide gel electrophoresis method, the methods utilizing charge differences, such as the ion exchange chromatography method, the methods utilizing specific affinities, such as the affinity chromatography method, the methods utilizing hydrophobicity differences, such as the reversed phase high performance liquid chromatography method, and the methods utilizing isoelectric point differences, such as the isoelectric focusing method, among others.

More specifically, contaminant nucleic acids and acidic proteins can be removed from the above-mentioned supernatant by subjecting said supernatant to ion exchange chromatography using DEAE-cellulose or the like. For example, it is efficient to apply the supernatant to a DEAE-cellulose column equilibrated with an almost neutral buffer (e.g. Tris buffer) and collect the effluent fraction. When said effluent fraction is subjected to ion exchange chromatography using CM-cellulose or the like, the hbFGF protein, which is a basic protein, is adsorbed on the carrier and can be eluted with a salt solution. CM-cellulose or the like acidic resin column chromatography can be used for the bacterial extract directly to purify hbFGF.

For example, it is efficient to apply the supernatant to a CM-cellulose column equilibrated with a slightly acidic buffer (such as phosphate buffer). After washing the column with the same buffer, hbFGF can be recovered by eluting the column with the buffer containing additional salts (such as NaCl). The eluate can be lyophilized after dialysis.

5

Affinity chromatography using heparin-Sepharose can be suitably applied to purifying hbFGF in Escherichia coli extracts. Thus, for instance, the hbFGF protein can be purified by applying the above eluate to a heparin-Sepharose column equilibrated with an almost neutral buffer (e.g. Tris or phosphate buffer), washing the column thoroughly and performing elution by linear gradient constructed with NaCl or the like.

Heparin columns (e.g. Shodex AF-pak HR-894, available from Showa Denko, Japan) developed for high performacen liquid chromatography are particularly efficient.

In this case, hbFGF can be recovered as homogeneous product in the same manner as in the case of the heparin-Sepharose column mentioned above, namely by applying the sample to a heparin column with an about neutral buffer, washing the column thoroughly and conducting elution on a linear gradient constructed with NaCl, for instance.

The thus-obtained product can be made up into a dry powder form by dialysis and lyophilization. To preserve the product with an added carrier (e.g. serum albumin) is desirable since the adsorption of the product on the vessel wall can be prevented thereby.

Furthermore, it is preferable to add a slight amount of a reducing agent in the course of purification or preservation, in order to prevent an oxidation of the product.

As examples of the reducing agent, there are mentioned $\beta$-mercaptoethanol, dithiothreitol, glutathione, and so forth.

In this way, substantially pure bhFGF can be obtained. The substantially pure hbFGF according to this invention includes products whose hbFGF protein content is not less than 95% (w/w) and, more preferably, products whose hbFGF content is not less than 98% (w/w).

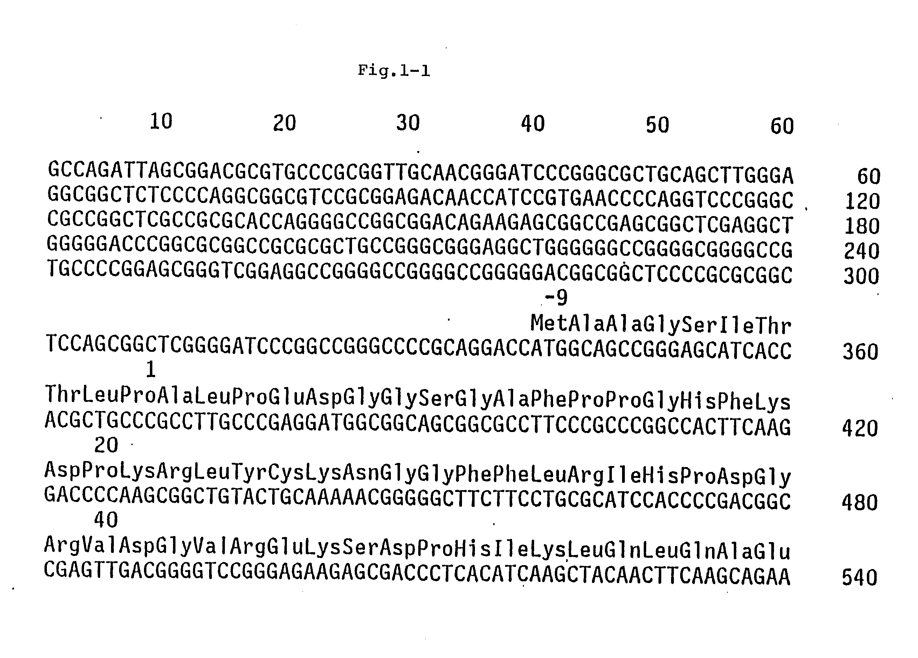

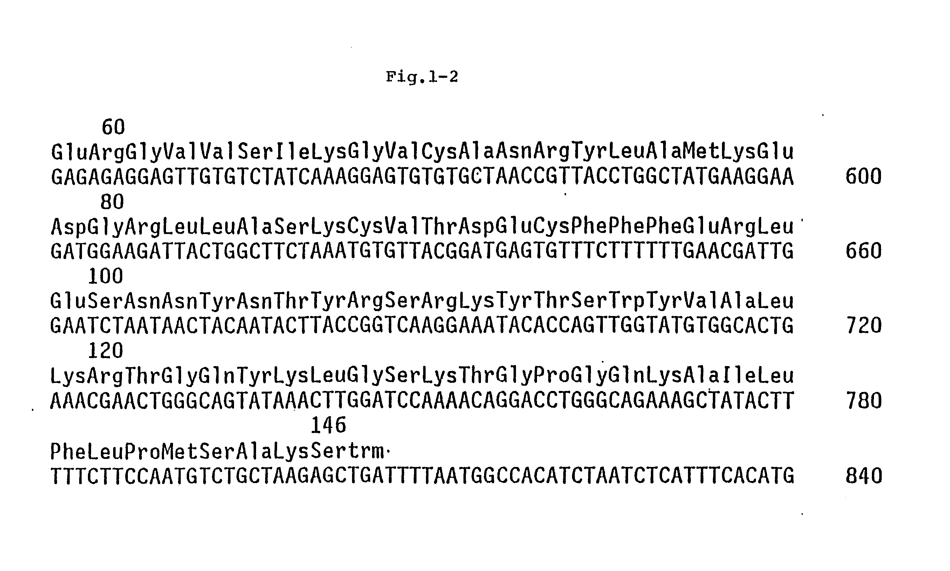

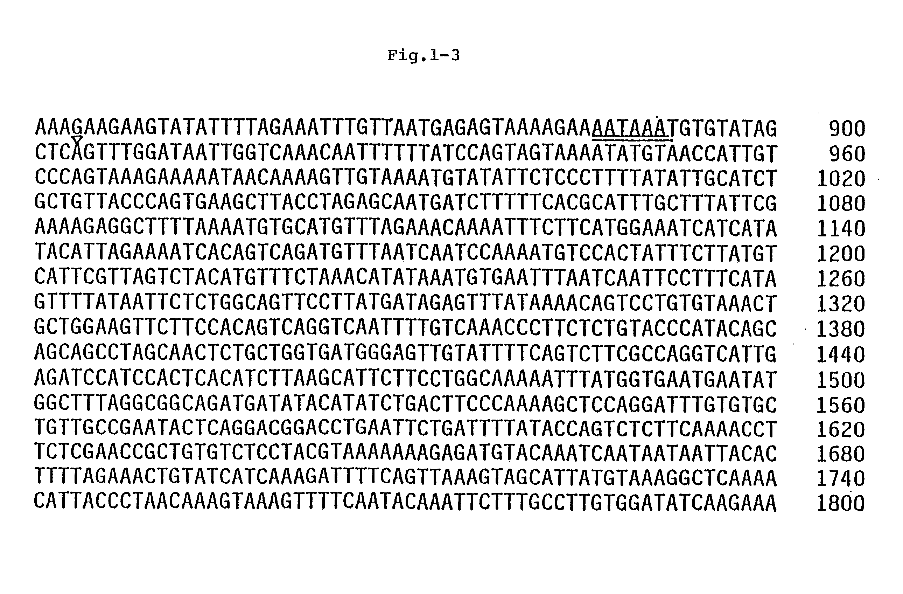

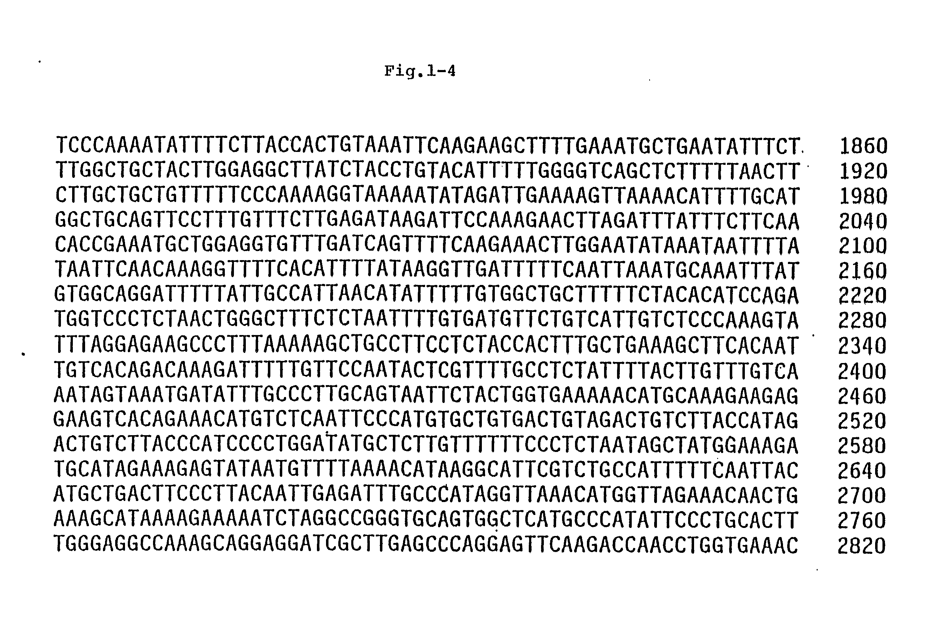

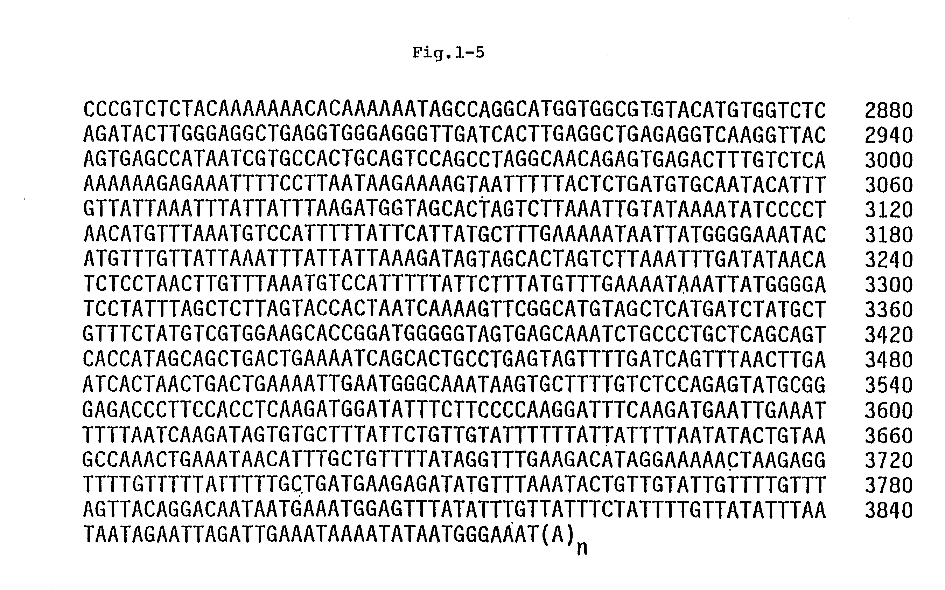

As an example of the hbFGF protein obtained by a genetic enginering technique according to this invention, there may be mentioned the protein including the polypeptide having the amino acid sequence shown in Fig. 1. Said polypeptide may have Met at the N-terminal thereof.

The activity of the thus-formed hbFGF can be assayed in terms of the BALB/c 3T3 cell growth promoting effect, for instance.

When gene-infected or transformed with the DNA according to the present invention, various cells in which, intrinsically, hbFGF cannot be synthesized or can be synthesized only in slight amounts, can allow synthesis of hbFGF in large amounts and thus can induce hbFGF advantageously.

When introduced into various cells, the expression plasmid containing the cDNA coding for the hbFGF protein according to the invention can make said cells to produce hbFGF, so that hbFGF can be obtained in large quantities. The so-produced hbFGF exhibits potent cell growth promoting action, and its toxicity is low. Therefore, the hbFGF can be used as a cure promoter for burns, wounds, postoperative tissues, etc., or as a therapeutic agent for thrombosis, arteriosclerosis, etc. which is based on its vascularizing effect. Furthermore, it can be used as a reagent for promoting cell cultivation.

For its pharmaceutical use, the hbFGF product according to the present invention can be safely administered to warm-blooded animals (e.g. human, mouse, rat, hamster, rabbit, dog, cat) parenterally or orally either per se in a powder form or in the form of pharmaceutical compositions (e.g. injection, tablet, capsule, solution, ointment) made up together with pharmacologically acceptable carriers, excipients and/or diluents.

Injectable preparations can be produced by a conventional method using, for example, physiological saline or an aqueous solution containing glucose and/or other adjuvant or adjuvants. Tablets, capsules and other pharmaceutical compositions can be prepared as well by a conventional method.

When hbFGF according to the present invention is used as a reagent for promoting cell culture, the hbFGF is added preferably in an amount of 0.01 to 10 $\mu$g, more preferably 0.1 to 10 $\mu$g, per liter of medium.

When hbFGF of the present invention is used as the cure promotor or the therapeutic agent, the amount of the hbFGF to be administered to the warm-blooded animals is small, and an appropriate amount is selected from 1 ng to 100 $\mu$g/kg a day according to the route of administration or symptoms.

hbFGF can be produced in large quantitites by cultivating the transformants according to the present invention in a medium, so that hbFGF which is useful as a drug and the like can be supplied in large quantities.

In the specification, claims and drawings, the abbreviations used for bases, amino acids and so on are those recommended by the IUPAC-IUB Commission on Biochemical Nomenclature or those conventionally used in the art. Examples are given below. Amino acids for which optical isomerism is possible are, unless otherwise specified, in the L form. DNA : Deoxyribonucleic acid

cDNA : Complementary deoxyribonucleic acid

A : Adenine

T : Thymine

G : Guanine

C : Cytosine

RNA : Ribonucleic acid

dATP : Deoxyadenosine triphosphate

dTTP : Deoxythymidine triphosphate

dGTP : Deoxyguanosine triphosphate

dCTP : Deoxycytidine triphsophate

ATP : Adenosine triphosphate

Tdr : Thymidine

EDTA : Ethyelenediaminetetraacetic acid

SDS : Sodium dodecyl sulfate

Gly : Glycine

Ala : Alanine

Val : Valine

Leu : Leucine

Ile : Isoleucine

Ser : Serine

Thr : Threonine

Cys : Cysteine

Met : Methionine

Glu : Glutamic acid

Asp : Aspartic acid

Lys : Lysine

Arg : Arginine

His : Histidine

Phe : Phenylalanine

Tyr : Tyrosine

Trp : Tryptophan

Pro : Proline

Asn : Asparagine

Gln : Glutamine

The transformant Escherichia coli K12 DH1/pTB 627 obtained in Example 1 (I) to be described later has been deposited at the Institute for Fermentation, Osaka (IFO), Japan since March 13, 1986 under the accession number of IFO 14494. This microorganism has also been deposited at the Fermentation Research Institute, Agency of Industrial Science and Technology, Ministry of International Trade and Industry (FRI), Japan since April 2, 1986 under the accession number of FERM P-8726, and the deposit has been converted to a deposit under the Budapest Treaty and the microorganism has been stored at FRI under the accession number of FERM BP-I280.

The transformant Escherichia coli K12 MM294/pTB669 obtained in Example 3 (1) to be described later has been deposited at the IFO since August 11, 1986 under the accession number IFO 14532. This microorganism has also been deposited at the FRI since August 21, 1986 under the accession number FERM P-8918, and the deposit has been converted to a deposit under the Budapest Treaty and the microorganism has been stored at FRI under the accession number of FERM BP-I28I.

The transformant mause L-bFGF-5 cell obtained in Example 5 (3) to be described later has been deposited at the IFO since July 30, 1986 under the deposit number of IFO 50092.

Brief Description of the Drawings

Fig. 1 shows the base sequence of the cDNA obtained in Example 1 (3) and the amino acid sequence estimable from said base sequence.

Fig. 2 shows the restriction enzyme map of the cDNA portion (about 4.3 kbp) and the vicinity thereof of the plasmid pTB627 obtained in Example I (5).

Fig. 3 shows the construction scheme for the plasmid pTB669 obtained in Example 3 (I).

Fig. 4 shows the construction scheme for the plasmid pTB 735 obtained in Example 4 (I).

Fig. 5 shows the construction scheme for the plasmid pTB663 obtained in Example 5 (I).

Fig. 6 shows the elution pattern from the HPLC-heparin column as obtained in Example 6.

Fig. 7 shows the SDS-polyacrylamide gel electrophoresis pattern of the peak I fraction as obtained in Example 6.

Fig. 8 shows the results of reversed phase chromatography as obtained in Example 7.

Fig. 9 shows the results of SDS-polyacrylamide gel electrophoresis as obtained in Example 7.

Fig. 10 shows the elution pattern of the CM-cellulose column chromatography as obtained in Example 8.

Fig. II shows the elution pattern of the HPLC-heparin column of the eluate of CM-cellulose colum chromatography, as obtained in Example 8.

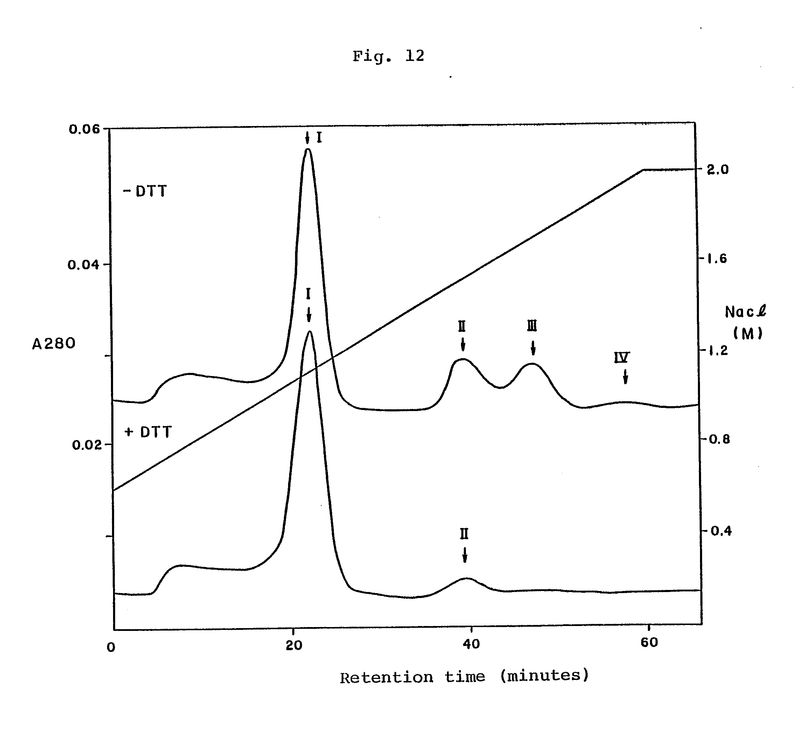

Fig. 12 shows the elution pattern of HPLC-heparin column as obtained in Example 10.

Examples

The following examples illustrate the present invention in further detail, but are by no means limitative of the present invention.

Example 1 (Construction of a plasmid)

(1) Isolation of cDNA-containing plasmid:

A cDNA library with Escherichia coli x1776 as the host as produced by inserting cDNA synthesized on the basis of human foreskin derived primary culture cell mRNA into the pCD vector [Okayama et al.: Molecular Cell Biology, 3, 280 (1983)] was given by Dr. Okayama at the National Institute of Child Health and Human Development, Bethesda, USA. The plasmid DNA was extracted from this cDNA library by the alkaline extraction method [Biorboim, H. C. & Doly, J.: Nucleic Acids Research, 1, 1513 (1979)] and Escherichia coli DH1 was infected with this DNA. A cDNA library was thus produced with Escherichia coli DH1 as the host. Said library comprised about 2 $\times$ 10⁶ clones.

The above cDNA library with Escherichia coli DH1 used therein was plated on 10 pieces of nitrocellulose filter (Millipore's HATF filter) in an amount of about 5 $\times$ 10⁴ clones per filter. Using these filters as master filters, 20 replica filters were prepared in 10 pairs corresponding to the master filters. Escherichia coli cells on these replica filters were lysed with 0.5 N NaOH and plasmid DNAs exposed and denatured were immobilized on the filters [Grunstein, M. & Hogness, D. S.: Proc. Natl. Acad. Sci. USA, 72, 3961 (1975)].

On the other hand, based on the amino acid sequence of bovine basic fibroblast growth factor as reported by F. Esch et al. [Proc. Natl. Acad. Sci. USA, 82, 6507 (1985)], base sequences corresponding to two amino acid sequences covering amino acids Nos. 13-20 (Pro-Pro-Gly-His-Phe-Lys-Asp-Pro) and amino acids Nos. 89-96 (Thr-Asp-Glu-Cys-Phe-Phe-Phe-Glu), respectively were chemically synthesized. (A unique nucleotide was selected at certain third positions at degenerated codons.) Thus, the base sequences synthesized were 5' GG$^A$/$_G$ TC$^T$/$_C$ TT$^A$ /$_G$ AA$^A$/$_G$ TGGCCAGGAGG and 5' TC$^A$/$_G$ AA$^A$/$_G$AA$^A$/$_G$ AA$^A$/$_G$ CA$^T$/$_C$ TC GTCGGT, each underlined base being one selected.) These oligonucleotides were labeled with ³²P at the 5' end by treating said oligonucleotides in 50 $\mu$l of reaction mixture [0.1 $\mu$g of oligonucleotide, 50 mM Tris-HCl, pH 8.0, 10 mM MgCl₂, 10 mM mercaptoethanol, 50 $\mu$Ci $\gamma$-³²P ATP (>5,000 Ci/mmole), 3 units of T4 polynucleotide kinase (Takara Shuzo, Japan)] at 37°C for 1 hour.

The thus-labeled oligonucleotides of the above two kinds were individually hybridized as probes with the replica filters. The hybridization reaction was conducted in 10 ml of a 100 $\mu$g/ml denatured salmon sperm DNA solution containing 10 $\mu$Ci of probe in 5 $\times$ SSPE [180 mM NaCl, 10 mM NaH₂PO₄, 1 mM EDTA (pH 7.4)] and 5 $\times$ Denhardt's with 0.1% SDS at 35°C for 16 hours. After reaction, the filters were washed with a 0.1% SDS solution in 5 $\times$ SSC [0.15 M NaCl, 0.015 M sodium citrate] three times each at room temperature for 30 minutes and then two times each at 45°C for 30 minutes [T. Maniatis et al.: "Molecular Cloning", Cold Spring Harbor Laboratory, page 309 (1982)].

Radioautograms were taken for the washed filters. A bacterial strain capable of reacting with the both kinds of probe was searched for by superposing the radioautograms for each pair of replica filters. In this manner, a strain [Escherichia coli K12 DHI/pTB627 (IFO 14494, FERM BP-1280)] capable of reacting with the two kinds of probe was obtained from among 5 $\times$ 10⁵ colonies.

(2) The plasmid DNA (pTB627) was extracted from the strain obtained above in (I) [Escherichia coli K12 DHI/pTB627 (IFO 14494, FERM BP-1280)] by the alkaline extraction method (vide supra) and purified. The cDNA portion of the plasmid DNA was excised by using the restriction enzyme BamHI (Takara Shuzo) and fractionated by agarose gel electrophoresis. The restriction enzyme BamHI cleaved the cDNA portion into 5 fragments (2.6 kbp, 650 bp, 430 bp, 250 bp and 150 bp, respectively). These DNA fragments were transferred from the agarose gel onto a nitrocellulose filter (S&S's BA 85) [Southern blotting method; T. Maniatis et al.: "Molecule Cloning", Cold Spring Harbor Laboratory, p. 382 (1982)]. This filter was hybridized with the above-mentioned two kinds of $^{32}$P-labeled oligonucleotide probe in the same manner as mentioned above. Radioautograms taken indicated that both kinds of probe had reacted with the 430 bp DNA fragment. Therefore, it was considered that the majority of the middle part of the amino acid sequence of human basic fibroblast growth factor (II) should be encoded on this 430 bp DNA fragment.

(3) Then, the base sequence of the cDNA portion mentioned above in (2), namely the five BamHI DNA fragments, was determined by the dideoxynucleotide synthetic chain termination method [J. Messing et al.: Nucleic Acids Research, 9, 309 (1981)].

Based on the results of sequencing, the whole amino acid sequence of human basic fibroblast growth factor (II) could be determined.

The base sequence of the cDNA and the amino acid sequence estimable from said base sequence are shown in Fig. 1. In Fig. I, the abbreviation "trm" stands for "terminater codon". The number n in $(A)_n$ is an arbitrary number. The amino acid sequence is very close to the amino acid sequence reported for bovine basic fibroblast growth factor. This suggests that said cDNA should be a human basic fibroblast growth factor (II)-encoding one.

In Fig. 1, the N-terminal amino acid (amino acid No. I Pro) was estimated from that of bovine basic fibroblast growth factor as reported by Esch et al. [Proc. Natl. Acad. Sci. USA, 82, 6507 (1985)].

The nine amino acids upstream from said N-terminal amino acid presumably constitute a signal-like peptide.

(4) The plasmid pTB627 obtained above in (2) was digested with BamHI and the 430 bp DNA fragment coding for human basic fibroblast growth factor was isolated. This DNA fragment was labeled with $^{32}$P by the nick translation method [Rigby, P. W. J. et al.: Journal of Molecular Biology, 113, 237 (1977)].

Hybridization reaction was carried out by using I0 filters with plasmid DNAs immobilized thereon as prepared by plating the cDNA library mentioned in Example I (I) with Escherichia coli DHI as the host on one hand and, on the other, the above $^{32}$P-labeled DNA fragment as a probe. The hybridization reaction was conducted in I0 ml of a 50% formamide solution containing I0 $\mu$Ci of thermally denatured probe (specific radioactivity 5 $\times$ I0$^7$ cpm/$\mu$g of DNA), 5 $\times$ SSPE (180 mM NaCl, I0 mM NaH$_2$PO$_4$, I mM EDTA, pH 7.4), 5 $\times$ Denhardt's, 0.1% SDS and 100 $\mu$g/ml denatured salmon sperm DNA at 42°C for I6 hours. After reaction, the filters were washed three times with 2 $\times$ SSC (0.I5 M NaCl, 0.015 M sodium citrate), 0.1% SDS solution at room temperature and further with 0.5 $\times$ SSC, 0.1% SDS solution at 60°C for 30 minutes.

Radioautograms were taken from the washed filters, and four strains reacting with the probe were picked up. The plasmid DNA was extracted from each of the strains by the alkaline extraction method (vide supra), purified and examined for the chain length of the cDNA portion. All the cDNA chain lengths obtained were as short as 500-700 bp. For the plasmid F7-I which was longest in chain length among the four plasmids, the base sequence of the cDNA portion was determined.

It was found that the F7-I cDNA begins with the middle of the codon for the I5th amino acid Gly - ($^{5'}$CCACTTC... ... ... ...), then enters, via the translational stop codon, the 3' untranslational region and ends after a poly(A) chain (about I50 bp) starting from a site (indicated by ∇ in Fig. I) about I00 bp downstream from the stop codon. Recognition of the poly(A) addition signal, namely the AATAAA sequence (doubly underlined in Fig. I), which is about 80 bp downstream from the translation stop codon, is considered to lead to addition of the poly(A) chain. Therefore it was shown that the human basic fibroblast growth factor mRNA may include multiple structures differing in the 3' untranslational region.

Furthermore, Northern blotting hybridization (Maniatis, T. et al.: Molecular Cloning, Cold Spring Harbor Laboratory, pp. 200, I982) for the mRNA extracted from human foreskin derived primary culture Flow 7000 cells (Flow U.S.A.) or human fetal lung-derived primary culture HEL cells with the above-mentioned $^{32}$P-labeled BamHI 430 bp fragment as a probe could detect at least five molecular species of mRNA.

(5) The restriction enzyme map of the cDNA portion (about 4.3 kbp) and the vicinity thereof of the plasmid pTB627 containing the cDNA for human basic fibroblast growth factor (II) is shown in Fig. 2. In Fig. 2, H, X, B, P and E indicate the HindIII, XhoI, BamHI, PstI and EcoRI cleavage sites, respectively.


Example 2 (Expression of hbFGF-encoding gene in animal cells)


Simian COS-7 cells were cultured in the manner of monolayer culture (Falcon plastic dish 60 mm in diameter) on DMEM medium containing I0% fetal calf serum and then the medium was replaced with a fresh portion of the same medium. Four hours after medium exchange, a calcium phosphate gel containing I0 $\mu$g of the DNA of the plasmid pTB627 was prepared by a known method [Graham et al.: Virology, 52 ,

456 (1973)] and added to the cells to give pTB627-infected COS-7 cells. Further 4 hours thereafter, the cells were treated with glycerol and then cultivation of the pTB627-infected COS-7 cells was continued on a medium containing 0.5% fetal calf serum. After 70-72 hours of cultivation, the medium containing hbFGF produced therein was collected.

Then, Falcon plastic dishes 35 mm in diameter were sown with BALB/c3T3 cells (2 × 10⁴ cells per dish) with DMEM medium containing 5% calf serum. On the next day, the medium was replaced with DMEM medium containing 0.5% calf serum. After the subsequent two-day cultivation, the above-mentioned hbFGF-containing medium was added to the dishes in 100 μl portions and, two days later, the cells were counted.

As shown in Table I, it was found that the medium used for cultivating pTB627-infected COS-7 cells contained hbFGF capable of inducing cell division of resting BALB/c 3T3 cells.

On the contrary, in a control experiment in which the medium used for uninfected cells was used, no hbFGF production was detected at all. The purified bovine brain-derived FGF was purchased from Takara Shuzo, Japan.

### Table I   hbFGF production by transfection with plasmid pTB627 DNA

| Sample added to medium for BALB/c3T3 cells | Number of cells per dish |
|---|---|
| Medium obtained by cultivating pTB627-infected COS-7 cells | $2.1 \times 10^5$ |
| Medium obtained by cultivating uninfected cells | $1.2 \times 10^5$ |
| Bovine brain FGF (0.2 ng/ml) | $1.9 \times 10^5$ |

Example 3 (Expression of hbFGF-encoding gene in Escherichia coli , in which trp promoter is used.)

(I) Construction of hbFGF expression plasmid pTB669

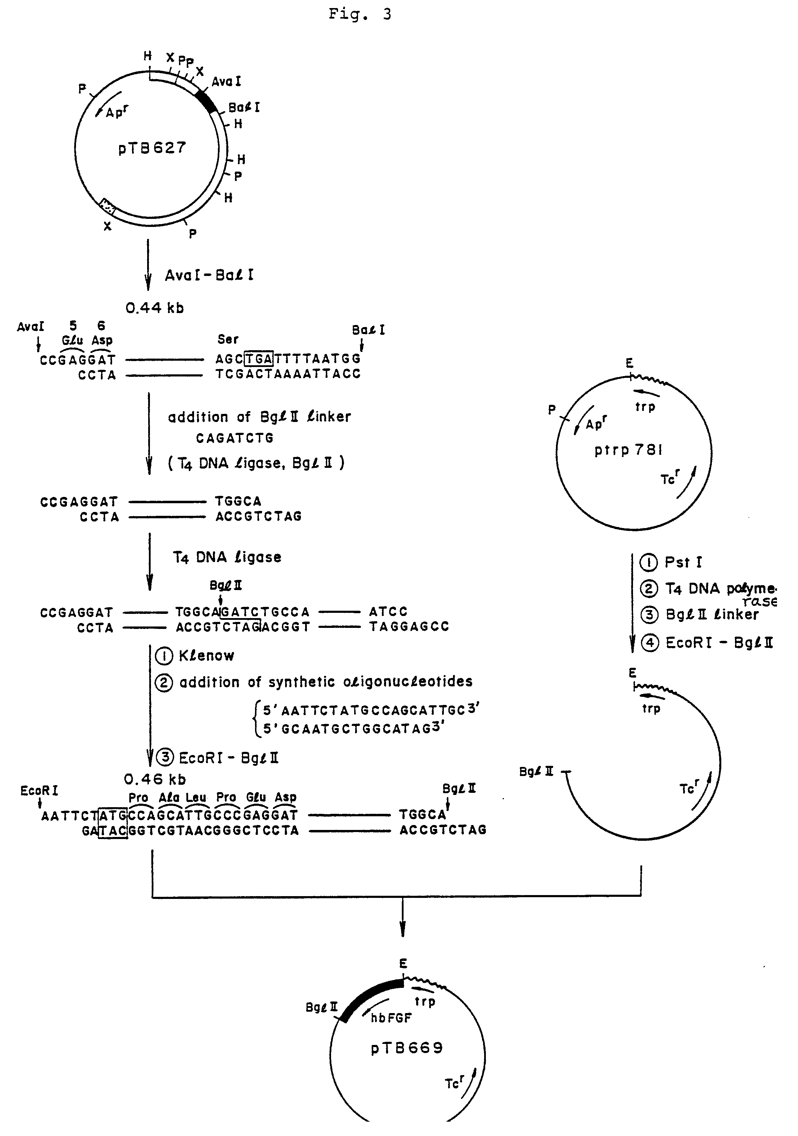

The plasmid pTB627 obtained in Example I (2) mentioned above and containing the hbFGF cDNA was cleaved with the restriction enzymes AvaI and BalI, whereby a 0.44 kb DNA fragment containing the hbFGF-encoding region. A BglII linker, pCAGATCTG, was ligated with that DNA at its BalI cleavage site (blunt end) by T₄-DNA ligase, and after digestion with Bgl II, a 0.44 kb AvaI-BglII DNA fragment was isolated. T₄ DNA ligase was allowed to act on this 0.44 kb AvaI-BglII fragment to thereby cause ligation between the BglII cleavage sites. Then, DNA polymerase (Klenow fragment) reaction was carried out in the presence of dXTPs to render the AvaI cleavage sites blunt. This DNA fragment was ligated with phsophorylated synthetic oligonucleotides, 5'AATTCTATGCCAGCATTGC3' and 5'GCAATGCTGGCATAG3', in the presence of T₄ DNA ligase. An about 0.46 kb DNA fragment was then prepared by cleavage with EcoRI-BglII. Separately, the trp promoter-containing plasmid ptrp781 [Kurokawa, T. et al.: Nucleic Acids Res., 11, 3077-3085 (1983)] was cleaved with PstI and rendered blunt-ended by T₄ DNA polymerase reaction. The BglII linker pCAGATCTG was joined to the above cleavage product at the blunt ends thereof by T4 DNA ligase reaction, the ligation product was cleaved with EcoRI-BglII and an about 3.2 kb DNA fragment containing the trp promoter, the tetracycline resistance gene and the plasmid replication origin was isolated. This 3.2 kb DNA fragment was ligated with the above-mentioned 0.46 kb EcoRI-BglII DNA fragment contianing the hbFGF-encoding gene region by T4 DNA ligase reaction, whereby an hbFGF expression plasmid, pTB669, was constructed (Fig. 3).

This plasmid pTB669 was used to transform Escherichia coli DH1 to give a transformant carrying the plasmid pTB669, namely Escherichia coli DH1/pTB669.

pTB669 was also used in the same manner to transform the Escherichia coli strains K12 MM294 and C600 to give Escherichia coli K12 MM294/pTB669 (IFO 14532, FERM BP-1281 and E. coli C600/pTB669, respectively.


## (2) Preparation of cell extract

Each of the above transformants was cultivated in M9 medium containing 1% glucose, 0.4% casamino acids and 8 μg/ml tetracycline. When the Klett value was about 200, 3-β-indolylacrylic acid was added to 25 μg/ml, and the cultivation was continued for further 4 hours. Thereafter, cells were harvested and suspended in one twentieth volume of 10% sucrose solution in 20 mM Tris-HCl, pH 7.6. To this suspension were added phenylmethylsulfonyl fluoride (PMSF) to 1 mM (final concentration), EDTA to 10 mM, NaCl to 0.1 M, spermidine hydrochloride to 10 mM and lysozyme to 100 μg/ml. After allowing to stand at 0°C for 45 minutes, the whole mixture was sonicated for 30 seconds. The sonication product was centrifuged at 18,000 rpm (Servall centrifuge, SS 34 roter, U.S.A.) for 30 minutes to give a supernatant, which was used as the cell extract.


## (3) hbFGF activity of cell extract

The hbFGF activity was expressed in terms of that weight of a purified bovine brain FGF reference standard (Takara Shuzo) which shows the same level of growth promoting activity against BALB/c3T3 cells as the sample.

A Nunc 96-well microtiter plate (flat bottomed) was sown with mouse BALB/c3T3 cells ($2 \times 10^3$ cells per well) with DMEM medium containing 5% calf serum (0.2 ml per well) and the cells were cultured. On the next day, the medium was replaced with DMEM medium containing 0.5% calf serum. After 3 days of cultivation, dilutions of the cell extract as prepared by serial 5-fold dilution with DME medium containing 0.5% BSA were added in an amount of 10 μl per well. After 20 hours of continued cultivation, 2 μl of $^3$H-Tdr (5 Ci/mmol, 0.5 mCi/ml RCC Amersham) was added to each well. Six hours later, cells in each well were scraped off by treatment with phosphate buffer (PBS) containing 0.2% trypsin and 0.02% EDTA and collected on a glass filter using a Titertek cell harvester and the quantity of $^3$H-Tdr taken up by the cells was measured using a scintillation counter. The activities of known amounts of bovine brain FGF (Takara Shuzo) were determined by following the same procedure. Based on the working curve thus obtained, the hbFGF contents in the samples were calculated. The results obtained are shown in Table 2.

In a control run, the hbFGF production in the transformant E. coli DH1/ptrp781 obtained by transformation of E. coli DHI with the plasmid ptrp781 was determined.


Table 2   hbFGF production

| Transformant | hbFGF production (on the per-liter-of-culture basis) |
|---|---|
| E. coli DH1/pTB669 | 2.95 mg |
| E. coli MM294/pTB669 | 23.15 |
| E. coli C600/pTB669 | 8.15 |
| E. coli DH1/ptrp781 | < 0.0005 |


Example 4 (Expression of hbFGF-encoding gene in Escherichia coli, in which λPL promoter is used.)

(I) Construction of hbFGF expression plasmid pTB 735:

The plasmid pTB 669 obtained in Example 3 (I) was cleaved with the restriction enzymes EcoRI and BglII, whereby a 0.46 kb DNA fragment was obtained. This fragment has an initiation codon ATG and the hbFGF-encoding sequences.

On the other hand, a plasmid pTB 28I [A plasmid having λ phage PL promoter. see Example 5 of European Patent Publication No. I779I5 A2.] was cleaved with Eco RI and Bam HI, whereby a DNA fragment, which has λ PL promoter, ampicilin-resistant gene and replication origin was isolated.

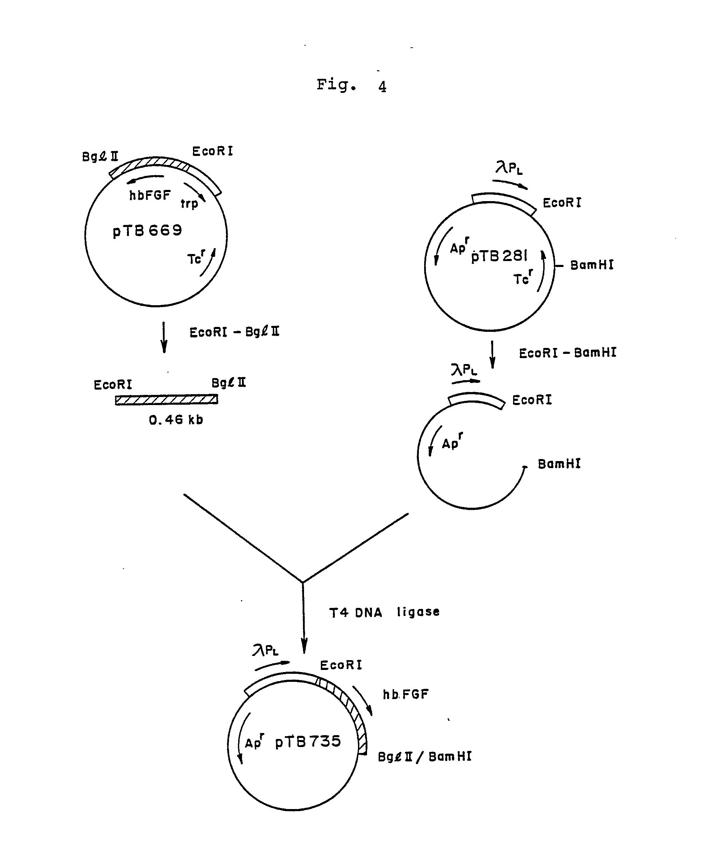

This DNA fragment was ligated with the above mentioned 0.46 Kb EcoRI-Bgl II DNA fragment by T4 DNA ligase, whereby an hbFGF expression plasmid pTB 735 was constructed (Fig. 4).

This plasmid pTB 735 was used to transform Escherichia coli N4830 cl 857(ts) to give a transformant carrying the plasmid pTB 735, namely Escherichia coli N4830 cl857(ts)/pTB735.

(2). Preparation of cell extract and its mitogenic activity:

The transformant obtained in the above item (I) was cultured in M9 medium that used in Example 3 (2) at 35°C.

When the Kett value of the medium reached about 200, the incubation temperature was shifted up to 42°C, and the culture was continued for another 2 hours.

After the cultivation, the cells were harvested, and cell extract was made in a similar manner as that of Example 3(2).

Namely the cells were suspended in one twentieth volume of I0% sucrose solution in 20 mM Tris•Hcl, pH 7.6, and to this suspension were added PMSF to I mM, EDTA to I0 mM, NaCl to 0.2 M, and lysozyme to I00 μg/ml.

After standing at 0°C for 45 minutes, the mixture was sonicated for 30 seconds. The sonicated mixture was centrifuged at I8000 rpm (Servall centrifuge, SS 34 roter, U.S.A.) for 30 minutes to give a supernatant, which was used as the cell extract.

The mitogenic activity of this cell extract was measured against BALB/c3T3 cells in accordance with the method of Example 3 (3), whereby the value as the same as that of Escherichia coli KI2 MM 294/pTB 669 was obtained.

Example 5 (Production of hbFGF-producing animal cell transformant)

(I) Construction of plasmid pTB663

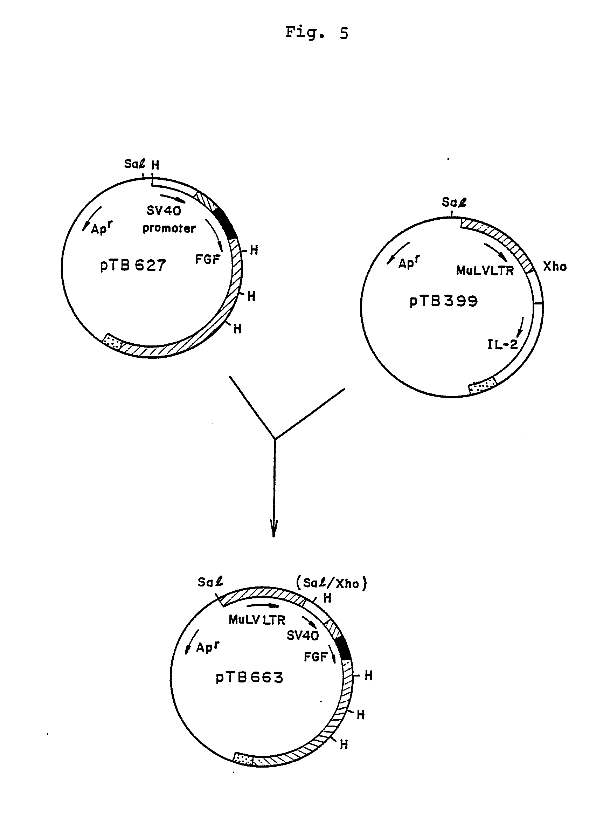

The plasmid pTB627 obtained in Example I (2) and containing the hbFGF-encoding gene was cleaved with the restriction enzyme Sall. Separately, the plasmid pTB399 (Japanese Patent Applicatin Laid-open No. 6I-63282, which corresponds to European Patent Publication No. I726I9) was cleaved with Sall-Xhol and a I.I kb DNA fragment containing the Abelson Murine Leukemia Virus (A-MuLV) long terminal repeat (LTR) region was isolated and purified. Both of these were mixed and ligated together by T4 DNA ligase reaction - (Sall cleavage and Xhol cleavage gave homologous cohesive ends) and a recombinant plasmid resulting from insertion of the I.I kb Sall-Xhol DNA fragment into pTB627 at the Sall cleavage site thereof with the LTR residing on the inserted I.I kb DNA fragment having the same directionality as that of the SV40 early promoter occurring upstream from the hbFGF gene was selected and named pTB663 (Fig. 5).

(2) Transformation of mouse L cells

Eagle's MEM medium containing I0% bovine fetal serum was placed in Falcon dishes (6 cm in diameter) and mouse TK (thymidine kinase)-deficient L cells (LTK⁻ strain) were cultured overnight at 37°C. After cultivation, these cells (7 × I0⁵ cells/dish) were cotransfected with 0.2 μg of the plasmid pTK61 - (Japanese Patent Application Laid-open No. 6I-63282 which corresponds to E.P. Publication No. I726I9; containing the TK gene of HSV) and I0 μg of pTB663 by the method of Graham et al. [Virology, 52, 456-467 (1973)]. After 4 hours of cultivation at 37°C, the medium was replaced with a fresh portion of the same medium and cultivation was continued overnight. On the next day, the medium was replaced with HAT medium (MEM medium containing l5 μg/ml hypoxanthine, l μg/ml aminopterine, 5 μg/ml thymidine and 0.25 μg/ml glycine) and cultivation was further continued at 37°C, with medium exchange at 3-or 4-day intervals. In about 2 to 3 weeks, cells converted to TK+ grew to form colonies.

(3) Cloning of transformant and assay of hbFGF

The transformant cells obtained in Example 5 (2) were cloned by the limited dilution method, whereby the transformant mouse cell lines L-bFGF-5 (IFO 50092) and L-bFGF-6 were obtained. The cloned cells after completion of cloning were cultured in Eagle's MEM medium containing 10% bovine fetal serum. The cells of each clone isolated were sown into a Linbro dish and, when the cells reached a state of about 80% saturation, the medium was replaced with Eagle's MEM medium containing 0.5% bovine fetal serum. After 48 hours of continued cultivation, the culture supernatant was assayed for FGF activity. The FGF activity measurement was conducted in the same manner as in Example l (3). The results thus obtained are shown in Table 3.

## Table 3   hbFGF production

| Transformant (clone) | hbFGF production |
|---|---|
| L-bFGF-5 | 4.8  ng/ml |
| L-bFGF-6 | 3.5  ng/ml |
| LTK⁻ strain | <0.01 ng/ml |

(4) Transformation of mouse BALB/c 3T3 cells and characteristics of transformant

Mouse BALB/c 3T3 A3l cells were transformed in the same manner as in Example 5 (2) to give a transformant.

The amounts of the produced hbFGF secreted in the cultured medium and in the cell lysate of the transformed cells were measured in the manner of Example 3(3).

The results are shown in Table 4.

From the results, it is understood that a large amount of hbFGF is stored in the cells compared to the amount of it secreted to the medium.

These transformed cells showed the specific morphological changes as in the case of A3l cell to which a purified hbFGF was added.

Furthermore, these transformants formed colonies in a soft agar.

Table 4

| transformant | Amount of hbFGF (ng/dish*) | |
| --- | --- | --- |
| | medium | cell extract |
| A-bFGF-1 | <0.5 | 134 |
| A-bFGF-4 | 3.2 | 238 |
| A31 | <0.5 | <0.02 |

Note: The amount of hbFGF per dish of 9 cm diameter.

Example 6 (Purification of hbFGF)

Following the procedure described in Example 3 (2), E. coli K12 MM294/pTB669 (IFO 14532, FERM BP-1281) was cultivated and a cell extract was prepared. A 25-ml portion of this extract (as prepared from 500 ml of culture broth) was passed through a DEAE-cellulose (DE52, Whatman, England) column {diameter 2 × 10 cm) equilibrated with 0.2 M NaCl solution in 20 mM Tris-HCl, pH 7.6 to thereby remove nucleic acid components in the extract. The effluent from the column and the column washings resultant from washing with 0.2 M NaCl solution in 20 mM Tris-HCl, pH 7.6 were collected and combined (DEAE effluent fraction 44 ml).

A 14-ml portion of this fraction was applied to a high performance liquid chromatograph (Gilson, France) equipped with a heparin column Shodex AF-pak HR-894 (8 mm ID × 5 cm, Showa Denko, Japan). The column was washed with 20 mM Tris-HCl, pH 7.6 and then with 0.5 M NaCl solution in 20 mM Tris-HCl, pH 7.6. Thereafter, elution was performed on a linear gradient of 0.5-2 M NaCl in 20 mM Tris-HCl buffer, pH 7.6, (eluent volume 60 ml, flow rate 1.0 ml/min).

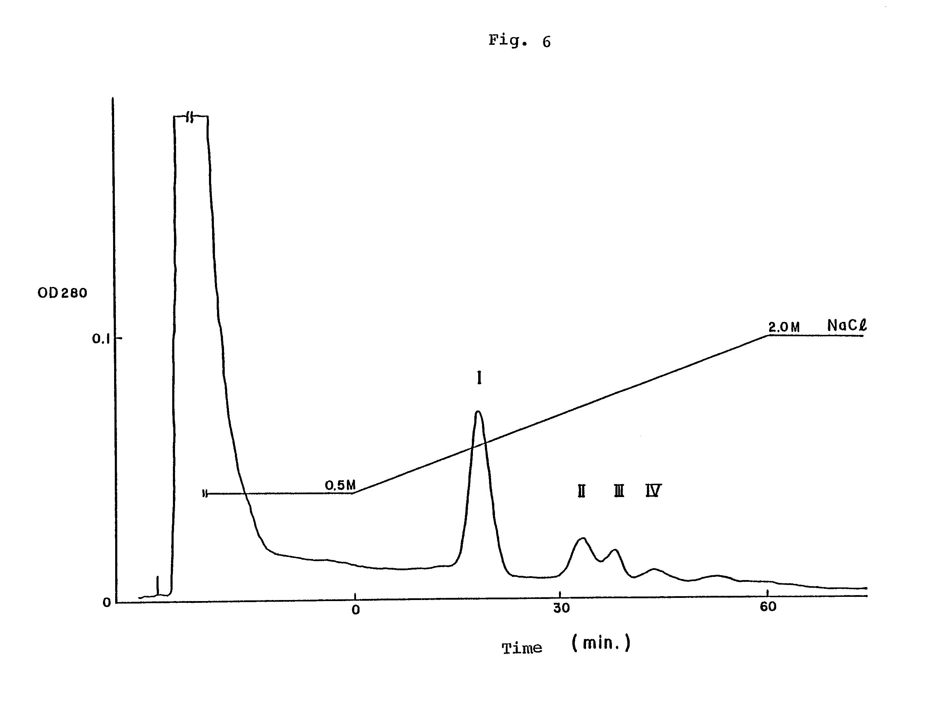

The elution pattern is shown in Fig. 6. In Fig. 6, the ordinate indicates the absorbance $OD_{280}$ as well as the NaCl concentration in the gradient. The abscissa indicates the time. At time 0, the gradient elution was started. Peak fractions were collected and examined for their FGF activity. The specific activity of the protein during this purification process and the hbFGF recoveries are shown in Table 5.

Table 5   Purification of hbFGF from recombinant
and specific activity

|  | Protein | Specific activity | Recovery (%) |
|---|---|---|---|
| Cell extract | 34.2 mg | 0.051 | 100 |
| DEAE-cellulose effluent fraction | 29.3 mg | 0.032 | 53.4 |
| Heparin column eluate fractions |  |  |  |
| Peak I | 200 µg | 2.9 | 33.0 |
| Peak II | 50 µg | 0.66 | 1.9 |
| Peak III | 27 µg | 1.03 | 1.6 |
| Peak IV | 10 µg | 0.34 | 0.2 |

In Table 5, the specific activity values are relative to the FGF activity (taken as 1) of bovine brain-derived FGF (Takara Shuzo; purity not less than 95%).

The BALB/c3T3 cell growth promoting activity was measured and the quantity of FGF which showed 50% activity relative to the maximum value was defined as I unit. Under these conditions, 0.02 ng of said Takara Shuzo's FGF showed an activity of I unit. (I ng = 50 units).

The peak I fraction showed the highest specific activity which was 2.9 times higher as compared with the commercial FGF (bovine brain-derived FGF, purity not less than 95%, Takara Shuzo). FGF activity was found also in peaks II, III and IV although the specific activity was low as compared with peak I.

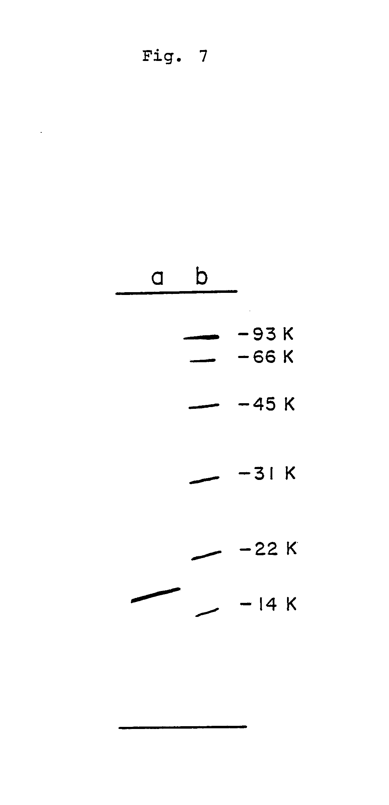

The peak I fraction was analyzed by 17.25% SDS polyacrylamide gel electrophoresis. A single band was observed at a position (molecular weight about 17,000) in good agreement with the molecular weight value calculated for the amino acid sequence of hbFGF (Fig. 7).

Peaks II, III and IV also gave a single band at the same position as in the case of peak I. The distribution among peak I, II and III is considered to be due to differeneces in steric structure of the protein.

In Fig. 7, the electrophoretic pattern a is for the peak I fraction and the pattern b is for molecular weight markers. The proteins were stained with Coumassie Brilliant Blue R250.

Example 7 (Characterization of hbFGF)

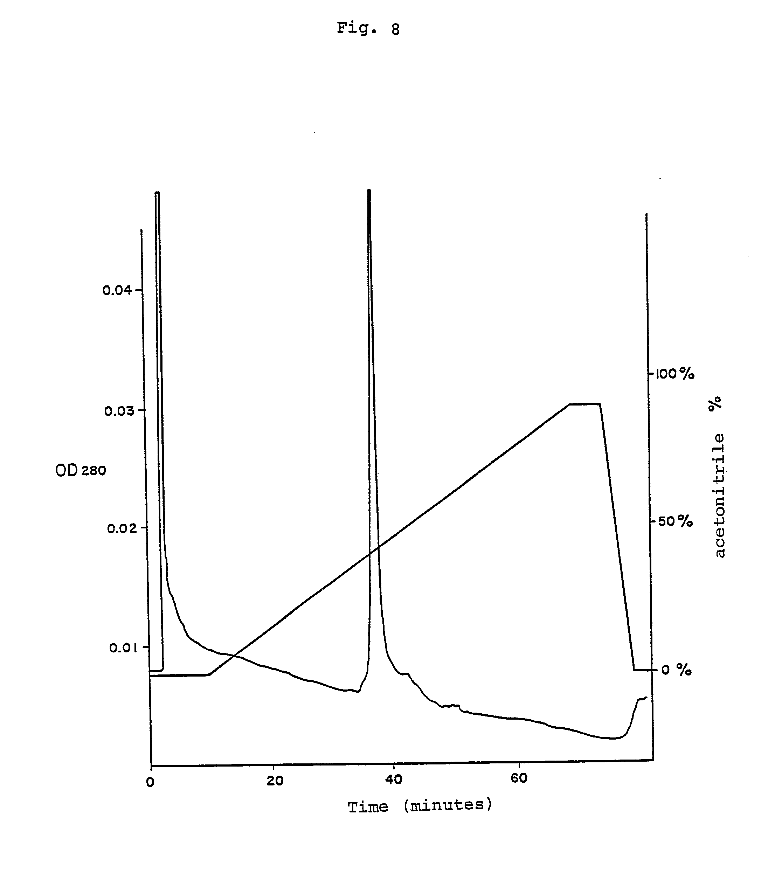

i) A 100-µg portion of the fraction (peak I) obtained by heparin affinity column chromatography in the above Example 5 was desalted by dialysis and then evaporated to dryness. The residue was suspended in 0.1% trifluoroacetic acid and analyzed by reversed phase chromatography (Chromatopacking Center, Ultron 300 C-4, Japan). The conditions of analysis were as follows: The rate of flow was I.0 ml/min. The solvent at the time of sample introduction was 0.1% trifluoroacetic acid. From I0 minutes thereafter, the acetonitrile concentration was increased linearly, so that, after 60 minutes, the solvent was composed of 90% acetonitrile and 0.1% trifluoroacetic acid. As a result, a peak was observed at about 40% acetonitrile, as shown in Fig 8.

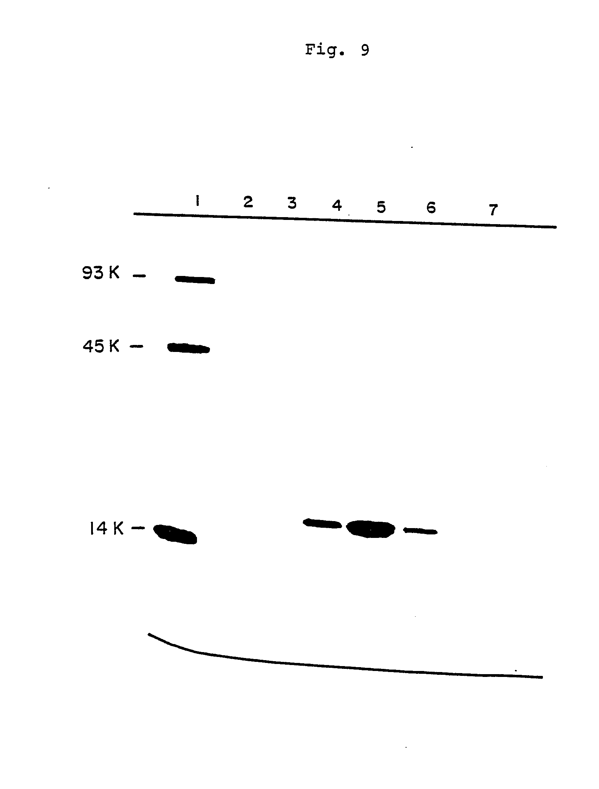

The eluate fractions around the peak as obtained in the above reversed phase chromatography were fractionated at one-minute intervals and subjected to polyacrylamide gel electrophoresis. The results thus obtained are shown in Fig. 9. In Fig. 9, the pattern I is for molecular weight markers, 2 for the 35th minute, 3 for the 36 minute, 4 for the 37th minute, 5 for the 38th minute, 6 for the 39th minute and 7 for the 40th minute eluate fraction.

These peak fractions (4, 5 and 6), when subjected to SDS-polyacrylamide gel electrophoresis, gave a single band. This pattern indicates that the hbFGF obtained in this way had a purity of 99%. The eluate fractions corresponding to these peaks were recovered and the product thus obtained was analyzed for amino acid composition and N-terminal amino acid sequence.

ii) Amino acid composition

The purified protein (14 μg) obtained in the above Example 7 (i) was placed in a glass test tube for hydrolysis, 200 volumes (v/w) of constant boiling point hydrochloric acid containing 4% thioglycolic acid was added, and the tube was sealed under reduced pressure. Hydrolysis was then conducted at 110°C for 24 hours. After hydrolysis, the tube was opened, the hydrochloric acid was removed under reuduced pressure, and the residue was dissolved in 0.02 N hydrochloric acid and analyzed for amino acids on a Hitachi model 835 high performance amino acid analyzer, Japan Cystine and cysteine were not assayed. Based on the molar ratios thus found, the number of residues was calculated for each amino acid assuming the number of aspartic acid residues as 12. The results thus obtained are shown in Table 6.

Table 6

| Amino acid detected | Number of residues | Amino acid composition* |
|---|---|---|
| Aspartic acid | 12.0 | 12 |
| Threonine | 4.8 | 5 |
| Serine | 8.3 | 10 |
| Glutamic acid | 11.8 | 12 |
| Proline | 8.5 | 9 |
| Glycine | 14.7 | 15 |
| Alanine | 8.9 | 9 |
| Cysteine | − | 4 |
| Valine | 6.4 | 7 |
| Methionine | 2.1 | 2 |
| Isoleucine | 3.8 | 4 |
| Leucine | 12.6 | 13 |
| Tyrosine | 6.9 | 7 |

| | | |
|---|---|---|
| Phenylalanine | 7.8 | 8 |
| Lysine | 14.3 | 14 |
| Histidine | 2.9 | 3 |
| Arginine | 10.8 | 11 |
| Tryptophan | 1.2 | 1 |

Note:  The numerical figures under "amino acid composition" in the above Table 5 are the theoretical amino acid composition values for hbFGF as estimated from the base sequence of the cDNA.

The amino acid residue numbers shown in Table 6 were in very good agreement with the amino acid composition of human basic fibroblast growth factor as estimated from the base sequence of the cDNA.

iii) Amino acid sequence of amino terminal portion

A 28-μg portion of the purified protein obtained in Example 7 (i) was analysed for the amino acid sequence of the N terminal portion using a vapor phase protein sequencer (Applied Biosystems Inc., model 470A, U.S.A.). The phenyltiohydantoin-amino acids (PTH-amino acids) formed were identified and assayed on a Varian high performance liquid chromatograph using a Micropak SP C-18-3 column (Varian, U.S.A.). The results thus obtained are shown in Table 7.

Table 7

| Cycle | PTH-amino acid (nmol) |
|---|---|
| 1 | Pro (0.49), Met (0.10) |
| 2 | Ala (0.51), Pro (0.05) |
| 3 | Leu (0.46), Ala (0.07) |
| 4 | Pro (0.33), Leu (0.09) |
| 5 | Glu (0.53), Pro (0.10) |

Based on the above results, the amino acid sequence of the amino terminal portion of the purified protein

was determined as follows: $\overset{1}{P}$ro-$\overset{2}{A}$la-$\overset{3}{L}$eu-$\overset{4}{P}$ro-$\overset{5}{G}$lu. This sequence was in agreement with that expected from the base sequence of the expression plasmid DNA.

Example 8 (Purification of hbFGF)

To 1 liter of a solution of 20 mM Tris•HClpH7.4, 1 mM EDTA, 0.2 M NaCl and 1 mM PMSF, 150 g of E-scherichia coli K12 MM294/pTB669 which has been cultivated in the manner of Example 3 was suspended.

This suspension is subjected to French press treatment at 4°C to give cell lysate.

This lysate was centrifuged at 18000 rpm at 4°C for 45 minutes with Serval centrifuge, SS 34 roter, to give 1.2 liters of a supernatant.

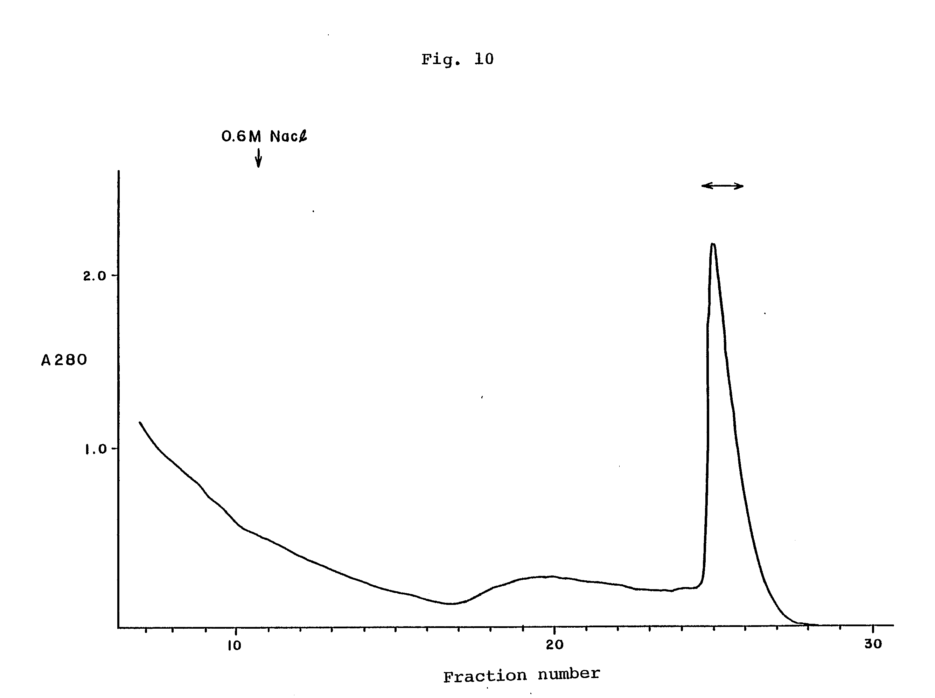

This supernatant was diluted with 0.1 M phosphate buffer, pH 6.0, to give 3 liters of solution, and the 2.4 liters of this solution were applied to a column of CM-cellulose (Whatmann, CM 50, diameter 5 cm × 30 cm). After washing the column with 0.1 M phosphate buffer, pH 6.0, an elution was carried out with 0.1 M phosphate buffer, pH 6.0 containing 0.6 M NaCl to give eluates of hbFGF. The absorbancy of the eluates at 280 nm were measured, and fractions which contain protein were collected. The elution pattern is shown in Fig. 10.

The pooled fraction which is marked ←→ in Fig. 10 was diluted threefold with 20 mM Tris•HCl pH 7.4, and then was applied to a high performance liquid chromatography (Gilson) equipped with a heparin column Shodex AF-pak HR-894 (8 mm ID × 25 cm, Showa Denko).

The CM-cellulose eluates were applied to the column through injection pump at a flow rate of 4 ml/min.

After washing the column with buffer A [20 mM Tris•HCl (pH 7.4) containing 1 mM EDTA.] at a flow rate of 6 ml/min., an elution was started under the following conditions:

The protein was eluted by linear gradient with the following buffers:

0 minutes: 70% buffer A, 30% buffer B;

200 minutes: 0% buffer A, 100% buffer B;

After 200 minutes: 100% buffer B

flow rate: 6 ml/min.

fraction: 4.5 ml each.

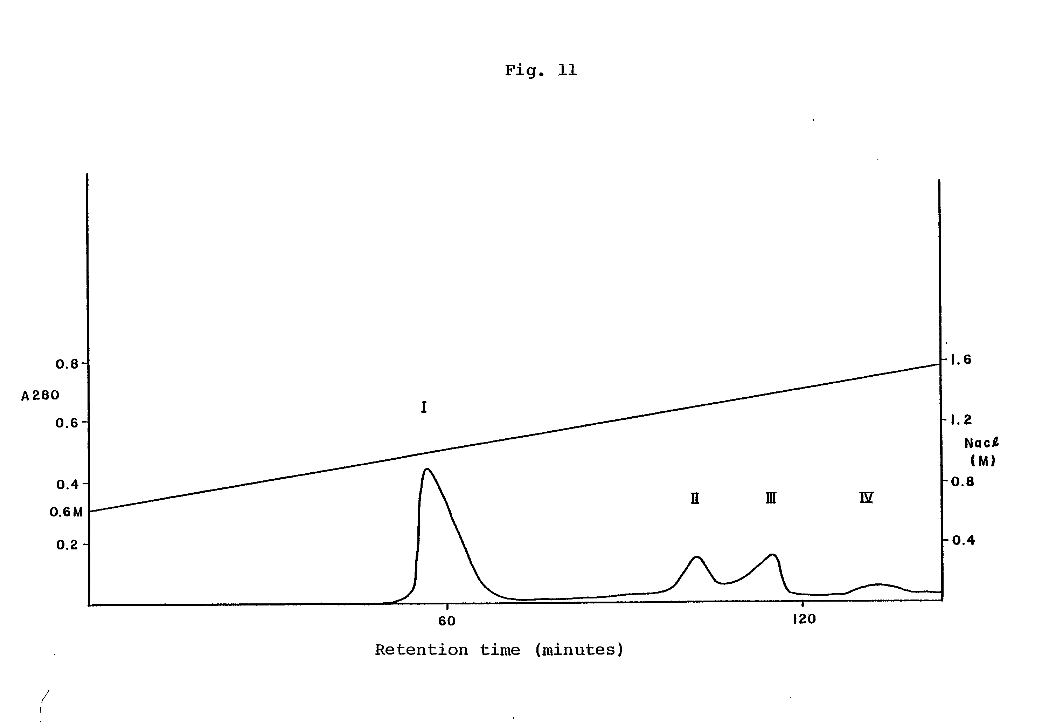

The elution pattern is shown in Fig. 11. In this chromatography, fractions of Peak I, Peak II, Peak III and Peak IV were obtained. By this procedure, about 20 mg of hbFGF was obtained.

Example 9 (Characterization of Peak II, III and IV)

Each about 100 µg of Peak II, Peak III and Peak IV fractions obtained by affinity chromatography using heparin column in Example 8, was dialysed and then lyophylized. The dried product was suspended in 0.1% trifluoroacetic acid and the suspension was applied to reverse phase chromatography. (Chromatopacking Center, Ultron 300 C-4, Japan). The elution conditions were the same as those of Example 6 (i). Namely, the rate of flow was 1.0 ml/min., and the solvent was 0.1% trifluoroacetic acid at the time of sample injection.

After 10 minutes, the acetonitrile concentration was increased linearly, so that, after 60 minutes, the solvent was composed of 90% acetonitrile and 0.1% trifluoroacetic acid.

The result showed that all of the three peaks, peak II, III and IV, were eluted as a sharp single peak at the same position of Peak I, which was eluted around 40% acetonitrile.

The peak fractions were recovered, and amino acid composition analysis and N-terminal amino acid sequence analysis were made in accordance with the method of Example 7 (ii), (iii). The results are shown in Tables 8 and 9.

The proteins of the peaks II, III and IV show no difference with peak I protein in terms of amino acid composition and N-terminal amino acid sequence, and are in consistent with those deduced from the base sequence of cDNA of expression plasmid.

Table 8

| Amino acid | Number of residues | | | Amino acid composition* |
|---|---|---|---|---|
| | Peak II | Peak III | Peak IV | |
| Asp & Asn | 12.0 | 12.0 | 12.0 | 12 |
| Thr | 4.7 | 4.7 | 4.8 | 5 |
| Ser | 8.6 | 8.5 | 8.5 | 10 |
| Glu & Gln | 12.6 | 12.5 | 12.4 | 12 |
| Pro | 9.3 | 9.2 | 9.2 | 9 |
| Gly | 14.8 | 14.6 | 14.7 | 5 |
| Ala | 9.2 | 9.1 | 9.3 | 9 |
| Cys* | - | - | - | 4 |
| Val | 6.5 | 6.7 | 7.0 | 7 |
| Met | 2.1 | 2.1 | 2.4 | 2 |
| Ile | 3.9 | 4.0 | 4.0 | 4 |
| Leu | 13.3 | 13.4 | 13.4 | 13 |
| Tyr | 7.1 | 7.3 | 7.3 | 7 |
| Phe | 8.2 | 8.4 | 8.5 | 8 |
| Lys | 13.6 | 13.8 | 14.0 | 14 |
| His | 2.8 | 3.0 | 3.2 | 3 |
| Arg | 11.0 | 11.0 | 11.2 | 11 |
| Trp | 0.9 | 1.2 | 1.6 | 1 |

Note:  The numerical figures under "amino acid composition" in the above Table 8 are the theoretical amino acid composition values for hbFGF estimated from the base sequence of the cDNA.

Table 9

| Cycle | PTH-amino acid (n mol*) | | |
|-------|--------|----------|---------|
|       | Peak II | Peak III | Peak IV |
| 1 | Pro (0.57) | Pro (1.87) | Pro (0.54) |
| 2 | Ala (0.89) | Ala (3.14) | Ala (0.97) |
| 3 | Lue (1.13) | Leu (3.84) | Leu (1.20) |
| 4 | Pro (0.52) | Pro (1.64) | Pro (0.52) |
| 5 | Glu (0.92) | Glu (3.21) | Glu (0.99) |

Note: The amount of the protein used for analysis was;
peak II, 43 μg; Peak III, 80 μg; Peak IV, 63 μg.

Example 10

Escherichia coli KI2 MM 294/pTB 669 (IFO I4532, FERM BP-I28I) was cultured in 500 ml of the medium the same as used in Example 3 (2).

The collected bacterial cells were suspended in 30 ml of 20 mM Tris•HCl (pH 7.6) containing 10% sucrose.

The suspension was made to the final concentration of 5 mM EDTA, 0.2 MNaCl, ImM PMSF, and was divided in two (A and B).

To one (B) of the suspension was added dithiothreitol (DTT) to the concentration of 0.5 mM.

To both the suspensions were added lysozyme to the concentration of I00 μg/ml, and the suspensions were kept standing at 0°C for 45 minutes. Then the suspensions were subjected to sonication.

These solutions were contrifuged at 20000 rpm (Serval centrifuge, SS-34 roter) for 40 minutes.

Each 7 ml of these cell extract (A) and (B) were separately applied to column chromatography of Shodex AF-pak HR-894 (8 mm ID × 5 cm).

After charging the sample, the column was washed with buffer A [0.6 M NaCl, 20 mM Tris•HCl (pH 7.4), I mM EDTA] until the absorption at 280 nm of eluate was reached to the background level.

Then elution was carried out by a linear gradient of buffer A to buffer B [2M NaCl, 20mM Tris•HCl i mM EDTA] at a flow rate of I ml/min (eluent volume 60 ml).

The elution pattern of the extract (A) and (B) is shown in Fig. I2.

From th eluton pattern of the extract (B) in which DTT was included, it could be seen that the almost all of the eluted protein was peak I and few other peaks (peak II, III and IV) were detected. It may be the effect of DTT which prevent protein denaturation caused by oxidation during purification steps.

So, this procedure brings high recovery of the most potent peak I fraction. The mitogenic activcity of the two peak I fractions obtained from the extract (A) and (B) was measured against BALB/c3T3 cell, and it was confirmed that both purified proteins had the same specific activity.

Claims

I. A human basic fibroblast growth actor which is a polypeptide including the amino acid sequence:
Pro-Ala-Leu-Pro-Glu-Asp-Gly-Gly-Ser-Gly-Ala-Phe-Pro-Pro-Gly-His-Phe-Lys-Asp-Pro-Lys-Arg-Leu-Tyr-Cys-
Lys-Asn-Gly-Gly-Phe-Phe-Leu-Arg-Ile-His-Pro-Asp-Gly-Arg-Val-Asp-Gly-Val-Arg-Glu-Lys-Ser-Asp-Pro-His-
Ile-Lys-Leu-Gln-Leu-Gln-Ala-Glu-Glu-Arg-Gly-Val-Val-Ser-Ile-Lys-Gly-Val-Cys-Ala-Asn-Arg-Try-Leu-Ala-Met-Lys-Glu-Asp-Gly-Arg-Leu-Leu-Ala-Ser-Lys-Cys-Val-Thr-Asp-Glu-Cys-Phe-Phe-Phe-Glu-Arg-Leu-Glu-Ser-Asn-
Asn-Tyr-Asn-Thr-Tyr-Arg-Ser-Arg-Lys-Tyr-Thr    -Ser-Trp-Tyr-Val-Ala-Leu-Lys-Arg-Thr-Gly-Gln-Tyr-Lys-Leu-
Gly-Ser-Lys-Thr-Gly-Pro-Gly-Gln-Lys-Ala-Ile-Leu-Phe-Leu-Pro-Met-Ser-Ala-Lys-Ser

2. A recombinant DNA, which contains a base sequence coding for the human basic fibroblast growth factor claimed in Claim I.

3. The DNA as claimed in Claim 2, wherein said base sequence includes the base sequence :

```
C C C G C C T T G C C C G A G G A T G G C G G C A G C G G C G C C T T C C C G C C C G G C C
A C T T C A A G G A C C C C A A G C G G C T G T A C T G C A A A A A C G G G G G C T T C T T C
C T G C G C A T C C A C C C C G A C G G C C G A G T T G A C G G G G T C C G G G A G A A G A
G C G A C C C T C A C A T C A A G C T A C A A C T T C A A G C A G A A G A G A G A G G A G T T
G T G T C T A T C A A A G G A G T G T G T G C T A A C C G T T A C C T G G C T A T G A A G G A
A G A T G G A A G A T T A C T G G C T T C T A A A T G T G T T A C G G A T G A G T G T T T C T T
T T T T G A A C G A T T G G A A T C T A A T A A C T A C A A T A C T T A C C G G T C A A G G A A
A T A C A C C A G T T G G T A T G T G G C A C T G A A A C G A A C T G G G C A G T A T A A A C T
T G G A T C C A A A A C A G G A C C T G G G C A G A A A G C T A T A C T T T T T C T T C C A A T
G T C T G C T A A G A G C
```

4. A transformant as transformed as a host cell with a vector including the DNA claimed in Claim 2.

5. The transformant as claimed in Claim 4, wherein the DNA is one claimed in Claim 3.

6. A method for producing a transformant, which comprises subjecting a host cell to transformation with a vector including the DNA claimed in Claim 2.

7. A method for producing the human basic fibroblast growth factor claimed in Claim I, which comprises cultivating the transformant claimed in Claim 4 in a medium and recovering the factor as produced and accumulated in the cultured broth.

8. A substantially pure human basic fibroblast growth factor (hbFGF) protein, the hbFGF being obtained by a genetic engineering technique.

9. The human basic fibroblast growth factor (hbFGF) protein as claimed in Claim 8, wherein the content of the hbFGF protein is not less than 95% (w/w).

I0. A method for producing a substantially pure human basic fibroblast growth factor (hbFGF) protein, which comprises subjecting a hbFGF which is obtained by a genetic engineering technique to a purification procedure of high performance liquid chromatography using a heparin column.

Claims for the Following Contract States AT,GR,ES

I. A method for producing a human basic fibroblast growth factor which is a polypeptide including the amino acid sequence :
Pro-Ala-Leu-Pro-Glu-Asp-Gly-Gly-Ser-Gly-Ala-Phe-Pro-Pro-Gly-His-Phe-Lys-Asp-Pro-Lys-Arg-Leu-Tyr-Cys-
Lys-Asn-Gly-Gly-Phe-Phe-Leu-Arg-Ile-His-Pro-Asp-Gly-Arg-Val-Asp-Gly-Val-Arg-Glu-Lys-Ser-Asp-Pro-His-
Ile-Lys-Leu-Gln-Leu-Gln-Ala-Glu-Glu-Arg-Gly-Val-Val-Ser-Ile-Lys-Gly-Val-Cys-Ala-Asn-Arg-Try-Leu-Ala-Met-
Lys-Glu-Asp-Gly-Arg-Leu-Leu-Ala-Ser-Lys-Cys-Val-Thr-Asp-Glu-Cys-Phe-Phe-Phe-Glu-Arg-Leu-Glu-Ser-Asn-
Asn-Tyr-Asn-Thr-Tyr-Arg-Ser-Arg-Lys-Tyr-Thr    -Ser-Trp-Tyr-Val-Ala-Leu-Lys-Arg-Thr-Gly-Gln-Tyr-Lys-Leu-
Gly-Ser-Lys-Thr-Gly-Pro-Gly-Gln-Lys-Ala-Ile-Leu-Phe-Leu-Pro-Met-Ser-Ala-Lys-Ser
which comprises cultivating a transformant transformed as a host cell with a vector including a DNA which contains a base sequence coding for the factor in a medium and recovering the factor as produced and accumulated in the cultured broth.

2. The method as claimed in Claim I, wherein the DNA includes the base sequence :

```
C C C G C C T T G C C C G A G G A T G G C G G C A G C G G C G C C T T C C C G C C C G G C C
A C T T C A A G G A C C C C A A G C G G C T G T A C T G C A A A A A C G G G G G C T T C T T C
C T G C G C A T C C A C C C C G A C G G C C G A G T T G A C G G G G T C C G G G A G A A G A
G C G A C C C T C A C A T C A A G C T A C A A C T T C A A G C A G A A G A G A G A G G A G T T
G T G T C T A T C A A A G G A G T G T G T G C T A A C C G T T A C C T G G C T A T G A A G G A
A G A T G G A A G A T T A C T G G C T T C T A A A T G T G T T A C G G A T G A G T G T T T C T T
T T T T G A A C G A T T G G A A T C T A A T A A C T A C A A T A C T T A C C G G T C A A G G A A
A T A C A C C A G T T G G T A T G T G G C A C T G A A A C G A A C T G G G C A G T A T A A A C T
T G G A T C C A A A A C A G G A C C T G G G C A G A A A G C T A T A C T T T T T C T T C C A A T
G T C T G C T A A G A G C
```

3. The method as claimed in Claim I, wherein the host cell in Escherichia coli.

21

4. The method as claimed in Claim I, wherein the transformant is <u>Escherichia coli</u> KI2 MM 294/pTB 669 (FERM BP-I28I).

5. A method for producing a transformant, which comprises subjecting a host cell to transformation with a vector including a DNA which contains a base sequence coding for the human basic fibroblast growth factor which is a polypeptide including the amino acid sequence: Pro-Ala-Leu-Pro-Glu-Asp-Gly-Gly-Ser-Gly-Ala-Phe-Pro-Pro-Gly-His-Phe-Lys-Asp-Pro-Lys-Arg-Leu-Tyr-Cys-Lys-Asn-Gly-Gly-Phe-Phe-Leu-Arg-Ile-His-Pro-Asp-Gly-Arg-Val-Asp-Gly-Val-Arg-Glu-Lys-Ser-Asp-Pro-His-Ile-Lys-Leu-Gln-Leu-Gln-Ala-Glu-Glu-Arg-Gly-Val-Val-Ser-Ile-Lys-Gly-Val-Cys-Ala-Asn-Arg-Try-Leu-Ala-Met-Lys-Glu-Asp-Gly-Arg-Leu-Leu-Ala-Ser-Lys-Cys-Val-Thr-Asp-Glu-Cys-Phe-Phe-Phe-Glu-Arg-Leu-Glu-Ser-Asn-Asn-Tyr-Asn-Thr-Tyr-Arg-Ser-Arg-Lys-Tyr-Thr-Ser-Trp-Tyr-Val-Ala-Leu-Lys-Arg-Thr-Gly-Gln-Tyr-Lys-Leu-Gly-Ser-Lys-Thr-Gly-Pro-Gly-Gln-Lys-Ala-Ile-Leu-Phe-Leu-Pro-Met-Ser-Ala-Lys-Ser

6. The method as claimed in Claim 5, wherein the host cell is <u>Escherichia coli</u>.

7. The method as claimed in Claim 5, wherein the DNA includes the base sequence :

C C C G C C T T G C C C G A G G A T G G C G G C A G C G G C G C C T T C C C G C C C G G C C
A C T T C A A G G A C C C C A A G C G G C T G T A C T G C A A A A A C G G G G G C T T C T T C
C T G C G C A T C C A C C C C G A C G G C C G A G T T G A C G G G G G T C C G G G A G A A G A
G C G A C C C T C A C A T C A A G C T A C A A C T T C A A G C A G A A G A G A G A G G A G T T
G T G T C T A T C A A A G G A G T G T G T G C T A A C C G T T A C C T G G C T A T G A A G G A
A G A T G G A A G A T T A C T G G C T T C T A A A T G T G T T A C G G A T G A G T G T T T C T T
T T T T G A A C G A T T G G A A T C T A A T A A C T A C A A T A C T T A C C G G T C A A G G A A
A T A C A C C A G T T G G T A T G T G G C A C T G A A A C G A A C T G G G C A G T A T A A A C T
T G G A T C C A A A A C A G G A C C T G G G C A G A A A G C T A T A C T T T T T C T T C C A A T
G T C T G C T A A G A G C

8. A method for producing a substantially pure human basic fibroblast growth factor (hbFGF) protein, which comprises subjecting a hbFGF which is obtained by a genetic engineering technique to a purification procedure of high performance liquid chromatography using a heparin column

9. The method as claimed in claim 8, wherein the content of the human basic fibroblast growth factor protein is not less than 95% (w/w).

I0. The method as claimed in Claim 8, wherein the content of the human basic fibroblast growth factor protein is not less than 98% (w/w).

Fig.1-1

```
           10           20           30           40           50           60
GCCAGATTAGCGGACGCGTGCCCGCGGTTGCAACGGGATCCCGGGCGCTGCAGCTTGGGA          60
GGCGGCTCTCCCCAGGCGGCGTCCGCGGAGACAACCATCCGTGAACCCCAGGTCCCGGGC         120
CGCCGGCTCGCCGCGCACCAGGGGCCGGCGGACAGAAGAGCGGCCGAGCGGCTCGAGGCT         180
GGGGGACCCGGCGCGGCCGCGCGCTGCCGGGCGGGAGGCTGGGGGGCCGGGGCGGGGCCG         240
TGCCCCGGAGCGGGTCGGAGGCCGGGGCCGGGGCCGGGGGACGGCGGCTCCCCGCGCGGC         300
                                          -9
                                          MetAlaAlaGlySerIleThr
TCCAGCGGCTCGGGGATCCCGGCCGGGCCCCGCAGGACCATGGCAGCCGGGAGCATCACC         360
     1
ThrLeuProAlaLeuProGluAspGlyGlySerGlyAlaPheProProGlyHisPheLys
ACGCTGCCCGCCTTGCCCGAGGATGGCGGCAGCGGCGCCTTCCCGCCCGGCCACTTCAAG         420
     20
AspProLysArgLeuTyrCysLysAsnGlyGlyPhePheLeuArgIleHisProAspGly
GACCCCAAGCGGCTGTACTGCAAAAACGGGGGCTTCTTCCTGCGCATCCACCCCGACGGC         480
     40
ArgValAspGlyValArgGluLysSerAspProHisIleLysLeuGlnLeuGlnAlaGlu
CGAGTTGACGGGGTCCGGGAGAAGAGCGACCCTCACATCAAGCTACAACTTCAAGCAGAA         540
```

Fig.1-2

60
GluArgGlyValValSerIleLysGlyValCysAlaAsnArgTyrLeuAlaMetLysGlu
GAGAGAGGAGTTGTGTCTATCAAAGGAGTGTGTGCTAACCGTTACCTGGCTATGAAGGAA          600
80
AspGlyArgLeuLeuAlaSerLysCysValThrAspGluCysPhePhePheGluArgLeu
GATGGAAGATTACTGGCTTCTAAATGTGTTACGGATGAGTGTTTCTTTTTTGAACGATTG          660
100
GluSerAsnAsnTyrAsnThrTyrArgSerArgLysTyrThrSerTrpTyrValAlaLeu
GAATCTAATAACTACAATACTTACCGGTCAAGGAAATACACCAGTTGGTATGTGGCACTG          720
120
LysArgThrGlyGlnTyrLysLeuGlySerLysThrGlyProGlyGlnLysAlaIleLeu
AAACGAACTGGGCAGTATAAACTTGGATCCAAAACAGGACCTGGGCAGAAAGCTATACTT          780
146
PheLeuProMetSerAlaLysSertrm·
TTTCTTCCAATGTCTGCTAAGAGCTGATTTTAATGGCCACATCTAATCTCATTTCACATG          840

0 237 966

Fig.1-3

```
AAAGAAGAAGTATATTTTAGAAATTTGTTAATGAGAGTAAAAGAAAATAAATGTGTATAG    900
    X
CTCAGTTTGGATAATTGGTCAAACAATTTTTTATCCAGTAGTAAAATATGTAACCATTGT    960
CCCAGTAAAGAAAAATAACAAAAGTTGTAAAATGTATATTCTCCCTTTTATATTGCATCT   1020
GCTGTTACCCAGTGAAGCTTACCTAGAGCAATGATCTTTTTCACGCATTTGCTTTATTCG   1080
AAAAGAGGCTTTTAAAATGTGCATGTTTAGAAACAAAATTTCTTCATGGAAATCATCATA   1140
TACATTAGAAAATCACAGTCAGATGTTTAATCAATCCAAAATGTCCACTATTTCTTATGT   1200
CATTCGTTAGTCTACATGTTTCTAAACATATAAATGTGAATTTAATCAATTCCTTTCATA   1260
GTTTTATAATTCTCTGGCAGTTCCTTATGATAGAGTTTATAAACAGTCCTGTGTAAACT   1320
GCTGGAAGTTCTTCCACAGTCAGGTCAATTTTGTCAAACCCTTCTCTGTACCCATACAGC   1380
AGCAGCCTAGCAACTCTGCTGGTGATGGGAGTTGTATTTTCAGTCTTCGCCAGGTCATTG   1440
AGATCCATCCACTCACATCTTAAGCATTCTTCCTGGCAAAAATTTATGGTGAATGAATAT   1500
GGCTTTAGGCGGCAGATGATATACATATCTGACTTCCCAAAAGCTCCAGGATTTGTGTGC   1560
TGTTGCCGAATACTCAGGACGGACCTGAATTCTGATTTTATACCAGTCTCTTCAAAACCT   1620
TCTCGAACCGCTGTGTCTCCTACGTAAAAAAGAGATGTACAAATCAATAATAATTACAC   1680
TTTTAGAAACTGTATCATCAAAGATTTTCAGTTAAAGTAGCATTATGTAAAGGCTCAAAA   1740
CATTACCCTAACAAAGTAAAGTTTTCAATACAAATTCTTTGCCTTGTGGATATCAAGAAA   1800
```

Fig.1-4

```
TCCCAAAATATTTTCTTACCACTGTAAATTCAAGAAGCTTTTGAAATGCTGAATATTTCT.    1860
TTGGCTGCTACTTGGAGGCTTATCTACCTGTACATTTTTGGGGTCAGCTCTTTTTAACTT    1920
CTTGCTGCTGTTTTTCCCAAAAGGTAAAAATATAGATTGAAAAGTTAAAACATTTTGCAT    1980
GGCTGCAGTTCCTTTGTTTCTTGAGATAAGATTCCAAAGAACTTAGATTTATTTCTTCAA    2040
CACCGAAATGCTGGAGGTGTTTGATCAGTTTTCAAGAAACTTGGAATATAAATAATTTTA    2100
TAATTCAACAAAGGTTTTCACATTTTATAAGGTTGATTTTTCAATTAAATGCAAATTTAT    2160
GTGGCAGGATTTTTATTGCCATTAACATATTTTTGTGGCTGCTTTTTCTACACATCCAGA    2220
TGGTCCCTCTAACTGGGCTTTCTCTAATTTTGTGATGTTCTGTCATTGTCTCCCAAAGTA    2280
TTTAGGAGAAGCCCTTTAAAAAGCTGCCTTCCTCTACCACTTTGCTGAAAGCTTCACAAT    2340
TGTCACAGACAAAGATTTTTGTTCCAATACTCGTTTTGCCTCTATTTTACTTGTTTGTCA    2400
AATAGTAAATGATATTTGCCCTTGCAGTAATTCTACTGGTGAAAAACATGCAAAGAAGAG    2460
GAAGTCACAGAAACATGTCTCAATTCCCATGTGCTGTGACTGTAGACTGTCTTACCATAG    2520
ACTGTCTTACCCATCCCCTGGATATGCTCTTGTTTTTTCCCTCTAATAGCTATGGAAAGA    2580
TGCATAGAAAGAGTATAATGTTTTAAAACATAAGGCATTCGTCTGCCATTTTTCAATTAC    2640
ATGCTGACTTCCCTTACAATTGAGATTTGCCCATAGGTTAAACATGGTTAGAAACAACTG    2700
AAAGCATAAAAGAAAAATCTAGGCCGGGTGCAGTGGCTCATGCCCATATTCCCTGCACTT    2760
TGGGAGGCCAAAGCAGGAGGATCGCTTGAGCCCAGGAGTTCAAGACCAACCTGGTGAAAC    2820
```

Fig.1-5

```
CCCGTCTCTACAAAAAAACACAAAAAATAGCCAGGCATGGTGGCGTGTACATGTGGTCTC    2880
AGATACTTGGGAGGCTGAGGTGGGAGGGTTGATCACTTGAGGCTGAGAGGTCAAGGTTAC    2940
AGTGAGCCATAATCGTGCCACTGCAGTCCAGCCTAGGCAACAGAGTGAGACTTTGTCTCA    3000
AAAAAAGAGAAATTTTCCTTAATAAGAAAAGTAATTTTTACTCTGATGTGCAATACATTT    3060
GTTATTAAATTTATTATTTAAGATGGTAGCACTAGTCTTAAATTGTATAAAATATCCCCT    3120
AACATGTTTAAATGTCCATTTTTATTCATTATGCTTTGAAAAATAATTATGGGGAAATAC    3180
ATGTTTGTTATTAAATTTATTATTAAAGATAGTAGCACTAGTCTTAAATTTGATATAACA    3240
TCTCCTAACTTGTTTAAATGTCCATTTTTATTCTTTATGTTTGAAAATAAATTATGGGGA    3300
TCCTATTTAGCTCTTAGTACCACTAATCAAAAGTTCGGCATGTAGCTCATGATCTATGCT    3360
GTTTCTATGTCGTGGAAGCACCGGATGGGGGTAGTGAGCAAATCTGCCCTGCTCAGCAGT    3420
CACCATAGCAGCTGACTGAAAATCAGCACTGCCTGAGTAGTTTTGATCAGTTTAACTTGA    3480
ATCACTAACTGACTGAAAATTGAATGGGCAAATAAGTGCTTTTGTCTCCAGAGTATGCGG    3540
GAGACCCTTCCACCTCAAGATGGATATTTCTTCCCCAAGGATTTCAAGATGAATTGAAAT    3600
TTTTAATCAAGATAGTGTGCTTTATTCTGTTGTATTTTTTATTATTTTAATATACTGTAA    3660
GCCAAACTGAAATAACATTTGCTGTTTTATAGGTTTGAAGACATAGGAAAAACTAAGAGG    3720
TTTTGTTTTTATTTTTGCTGATGAAGAGATATGTTTAAATACTGTTGTATTGTTTTGTTT    3780
AGTTACAGGACAATAATGAAATGGAGTTTATATTTGTTATTTCTATTTTGTTATATTTAA    3840
TAATAGAATTAGATTGAAATAAAATATAATGGGAAAT(A)$_n$
```

0 237 966

## Fig. 2

H: Hind III,   X: Xho I,   B: Bam HI,   P: Pst I

Fig. 3

Fig. 4

Fig. 5

Fig. 6

0 237 966

Fig. 7

Fig. 8

Fig. 9

| | 1 | 2 | 3 | 4 | 5 | 6 | 7 |
|---|---|---|---|---|---|---|---|

93 K —

45 K —

14 K —

Fig. 10

0 237 966

Fig. 11

Fig. 12